# EUROPEAN PATENT APPLICATION

(11) **EP 3 287 009 A1**
(43) Date of publication of application: **28.02.2018**
(21) Application number: 16185992.1
(22) Date of filing: 26.08.2016
(51) Int. Cl.: A01N 25/34, A01N 25/24, A01N 47/44, A01N 59/16, A01N 33/12, D06M 11/13, A61L 2/16, D06M 13/256, D06M 13/352, D06M 16/00, A62B 23/02, A41D 13/11

(54) **NON-LEACHING SURFACE SANITIZER AND WIPE WITH IMPROVED WASHABILITY AND/OR ABSORBENCY**

(71) Applicant: Green Impact Holding AG, 6330 Cham/Zug (CH)
(72) Inventor: SWAMY, Sanjeev, 6442 Gersau (CH)
(74) Representative: Karl, Christof

(57) **Abstract**

The present invention is directed to a textile material to which one or more antimicrobial and/or hydrophilic and/or stain release agents are adhered. The agent(s) is/are adhered to the textile material in such a manner that they are not released from the textile even if the textile is wetted or washed, so that the textile is reusable. In preferred embodiments, where one or more antimicrobial agents are adhered to the textile, the textile can be used to sanitize a surface in a non-leaching manner, e.g. without a liquid sanitizer, both under wet and dry conditions. Washability and/or usability of the textile are improved where one or more hydrophilic and/or stain release agents are adhered to the textile, which is particularly advantageous if the textile is used as a wipe or for similar purposes. The invention further relates to a method of finishing a textile material by applying and binding antimicrobial and/or hydrophilic and/or stain release agents to the textile material so that the agents are essentially irreversibly adhered to the finished textile material.

## Description

### FIELD OF THE INVENTION

The present invention is directed to a textile material to which one or more antimicrobial and/or hydrophilic and/or stain release agents are adhered. The agent(s) is/are adhered to the textile material in such a manner that they are not released from the textile even if the textile is wetted or washed, so that the textile is reusable. In preferred embodiments, where one or more antimicrobial agents are adhered to the textile, the textile can be used to sanitize a surface in a non-leaching manner, e.g. without a liquid sanitizer, both under wet and dry conditions. Washability and/or usability of the textile are improved where one or more hydrophilic and/or stain release agents are adhered to the textile, which is particularly advantageous if the textile is used as a wipe or for similar purposes. The invention further relates to a method of finishing a textile material by applying and binding antimicrobial and/or hydrophilic and/or stain release agents to the textile material so that the agents are essentially irreversibly adhered to the finished textile material.

### BACKGROUND OF THE INVENTION

Textile materials like fabrics, yarns and/or fibers are used for a variety of purposes and in a variety of environments, such as wiping and cleaning a surface. There is a realistic danger of microbiological contamination on the textile. Danger of staining due to ketchup, honey, sputum, blood, human excreta and moisture etc. are also problems in various circumstances. Not only do such stains look unpleasant, but they also are fertile breeding grounds for various harmful bacteria, fungi and viruses on the textile substrates. The inner surface of a textile, dead tissue, sweat, humidity and moisture aids the growth and spread of various pathogens. Such textiles should either be disposed of or washed if re-use is possible. Washing may lead to an excessive consumption of water, and large quantities of detergents may have to be used to launder the textiles. Both, the washing and the disposal are inefficient and costly.

Disinfection/sterilization is an important process in everyday life. It can be rated at various levels. There are various recordings of the requirements of the levels of performance which can be noted, for example, as per the United States National Pesticide Information Center, and under the link http://npic.orst.edu/factsheets/antimicrobials.html. A table therefrom, as can be taken hereinafter, shows that that agency distinguishes three main types of public health antimicrobial pesticides as follows.

| | Sanitizer | Disinfectant | Sterilizer |
|---|---|---|---|
| Effective against | 99.9% | 100% | 100% |
| | | | |
| | • Bacteria | • Bacteria | • Bacteria |
| | | • Fungi | • Fungi |
| | | • Certain | • Viruses |
| | | viruses | • Spores |
| | | | |
| Time required for effectiveness | 30 seconds to 5 minutes | Generally 10 minutes | Variable |
| Locations / Uses | Household surfaces Food contact surfaces | Household surfaces Medical settings | Medical instruments Research supplies |
| Effect | Limited microbicide | Microbicide Irreversible microbistat | Microbicide |

Current surface disinfectants available on the market work for the moment when applied or used, but are not re-usable or long lasting in nature. For example, when chlorhexidine is sprayed on a contaminated surface, it is consumed in that instant. Some conventional sanitizing wipes are impregnated with liquid antimicrobial agents which are leached or released onto the surface to be sanitized. However, since the antimicrobial agents are consumed during the use of the wipe, they can typically only be used once.

US patent application 2003/0157147 tries to overcome this problem by a long-lasting wipe using metallic silver as an oligodynamic agent. The wipe can be used, e.g., as an anti-bacterial kitchen wipe for sanitizing the food preparation areas while cleaning up general food debris for these areas. Studies of microbe reduction on the wipe were made, but not on the surface to be sanitized. The textile fabric is coated by depositing a metallic coating such as silver on a substrate, including chemical plating, plasma vapor deposition (PVD), chemical vapor deposition (CVD) and combustion chemical vapor deposition (CCVD). A silver coated outside layer contains preferably 3-25% silver o.w.f. Due to the extensive use of silver, the costs of the wipe are, however, excessively high. Furthermore, since the silver-coated outside layer is hydrophobic in nature, usability of the wipe for cleaning with water or other aqueous solvents is decreased, and it needs to be equipped with a hydrophilic inner layer, which further increases the manufacturing costs.

The website http://www.microclean.at (last access on 12 August 2016) also discloses wipes with integrated, antibacterial silver. It is claimed that a surface can largely be freed from pathogenic organisms such as bacteria and other germs by cleaning the surface using the wipes and water. The manufacturing process of the wipe is not disclosed. According to a report published on the website, embodiments of the wipe contain silver in an amount of 0.17% o.w.f. While this amount is much lower than the one in the wipes disclosed in US 2003/0157147, it is still high and makes the wipes expensive. In addition, surface sanitizing only seems to work in combination with the use of water. Finally, there is no indication of good wash-durability or improved washability or usability of the wipes.

US patent 4,395,454 discloses an absorbent, highly wettable, bioactive, dyed substrate having incorporated on it a bioactive silicone quaternary amine (organosilane), a hydrophilic agent (organosilicone terpolymer) and a direct dye. Applications for the material include various medical-type substrates. The document explains that when silicone quaternary amines are applied to a non-woven substrate, it was found that the substrate was rendered hydrophobic. Furthermore, the document claims that the silicone quaternary amine-type antimicrobial compounds do not readily adhere to polyester substrates. As a solution to the problem, a treating solution containing a specific silicone quaternary amine also contains organosilicone terpolymer, which acts as a hydrophilic coupling agent and rewetting agent, and a direct dye. The substrate is, however, not wash-durable and in some preferred embodiments not even re-usable.

### SUMMARY OF THE INVENTION

It is an object of the invention to provide textile materials that overcome some or all problems of the above-mentioned prior art documents. In particular, one object of the invention is to provide textile materials that can be used for sanitizing a surface in a non-leaching manner, both under wet and dry conditions. More generally is it an object of the invention that the textile materials can kill microbes or inhibit their growth as completely as possible, both on the surface to be sanitized and on the textile material itself. Furthermore, it is an object of the invention that the textile materials can be reused even after application and numerous washes. It is a further object of the invention that the textile materials are easily washable and/or well suitable for use as a hygiene product such as a wipe.

Some or all of these objects are solved by the subject matter of the independent claims. Preferred embodiments are subject of the dependent claims.

In accordance with the present invention, a textile material is provided to which one or more chemical agents are adhered, which chemical agents convey antimicrobial and/or hydrophilic and/or stain release properties to the textile material, in a wash-durable manner. The textile material can be used for hygiene products, e.g. as a wipe and/or surface sanitizer. The invention further provides finishing methods for manufacturing the textile materials, by applying liquor application processes such as padding and exhaust processes, followed by heat treatment.

Unless otherwise stated, all percentages hereinafter refer to the ratio of the uptaken weight of antimicrobial agent on a textile and the weight of that fabric without the uptaken antimicrobial agent. The term "on weight fabric" refers to this ratio. Abbreviations are "owf" or "o.w.f". The term "gpl" means "grams per liter" and is typically used to define the concentration of a substance in a liquor.

In the context of the present invention the terms "textile" and "textile material" relate to a flexible material consisting of fibres, or a network of natural and/or artificial fibres, such as a yarn or a fabric. The material may be in its natural or processed or even finished form. The term "starting textile material" refers to a textile material which has not yet been treated by the finishing processes described in the present disclosure.

The term "antimicrobial" as used in the context of the present invention relates to the ability to kill at least some types of microorganisms, or to inhibit the growth or reproduction of at least some types of microorganisms. Said term relates to any compound, agent, product or process that is harmful to one or more "microorganism" as used in the context of the present invention. Preferably, the one or more "microorganisms" get killed by the "antimicrobial" product or process. By "antimicrobial agent" is meant any substance or combination of substances that kills or prevents the growth of a microorganism. The terms "microorganism" and "microbe", which are used interchangeably in the context of the present invention, are defined to comprise any organism too small to be seen by the unaided eye, such as, especially, single-celled organisms. In particular, the terms "microorganism" and "microbe" cover prokaryotes including bacteria and archaea, eukaryotes including protists, animals like dust mites or spider mites, fungi, and plants like green algae, as well as viruses.

The term "hydrophilic" essentially relates to the attraction of water. In the context of the present invention, the term "hydrophilic" commonly implies attachment or adhering of aqueous liquids at the surface and in the interior of a textile material. Thus, the term "hydrophilic" or "hydrophilic properties" as used herein includes the attachment or adhering of aqueous liquids at or near the surface of a textile material and/or in the interior of a textile material. When the term "hydrophilic" or "hydrophilic properties" is used herein, it means the attachment or adhering of liquid at or near the surface of a textile material. A hydrophilic textile is typically not water-repellent and to the contrary absorbs water quickly. It typically also exhibits good wicking properties. A "hydrophilic agent" is an agent which when applied to a substrate like a textile material, noticeably improves the hydrophilic properties of the substrate.

The term "stain release" relates to easy removal of stains. In the context of the present invention, the term "stain release properties" of a textile commonly refers to its ability of both adsorbing in particular oily stains on the outside surface or on internal surfaces within a textile material and easily removing stains from these surfaces, for instance during laundry. The term "stain release properties" is not to be confused with the term "stain repellent properties" of a textile. Whereas textiles with good stain repellent properties do not adsorb or absorb oily stains in the first place, textiles with good stain release properties may adsorb them but subsequently release them easily. A "stain release agent" is an agent which, when applied to a substrate like a textile material, noticeably improves the stain release properties of that substrate.

Whenever a temperature is mentioned in the present specification, the temperature refers to a temperature applied at normal pressure (101.325 Pa). If in an implementation of the invention higher or lower pressure is applied, the temperatures are understood to be adapted accordingly.

A 1st embodiment of the invention is a textile material to which one or more antimicrobial agents are adhered.

According to a 2^{nd} embodiment, in the i^{st} embodiment, one or more hydrophilic agents are adhered to the textile.

According to a 3^{rd} embodiment, in the textile material according to the 1^{st} embodiment, has one or more stain release agents adhered.

A 4^{th} embodiment is the textile material according to both the 2^{nd} and the 3^{rd} embodiments.

### Antimicrobial agents

### Types

According to a 5^{th} embodiment, in the textile material according to any one of the preceding embodiments, the one or more antimicrobial agents adhered to the textile material comprise
- at least one, preferably at least two, more preferably at least three, even more preferably at least four, and most preferably all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably at least three, and most preferably all four selected from the group consisting of metal, an azole-based compound, a quaternary ammonium organosilane compound, and polyhexamethylene biguanide; or
- at least one, preferably at least two, more preferably all three selected from the group consisting of metal, an azole-based compound, and polyhexamethylene biguanide ; or
- at least one, preferably at least two, more preferably all three selected from the group consisting of a quaternary ammonium organosilane compound, metal, and an azole-based compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

According to a 6^{th} embodiment, in the textile material according to any one of the preceding embodiments 1 to 4, the one or more antimicrobial agents adhered to the textile material comprise
- polyhexamethylene biguanide and at least one, preferably both selected from the group consisting of metal and an azole-based compound; or
- metal and at least one, preferably both selected from the group consisting of polyhexamethylene biguanide and an azole-based compound; or
- an azole-based compound and at least one, preferably both selected from the group consisting of polyhexamethylene biguanide and metal; or
- quaternary ammonium organosilane and at least one, preferably both selected from the group consisting of metal and an azole-based compound; or
- metal and at least one, preferably both selected from the group consisting of a quaternary ammonium organosilane compound and an azole-based compound; or
- an azole-based compound and at least one, preferably both selected from the group consisting of quaternary ammonium organosilane and metal.

According to a 7^{th} embodiment, in the textile material according to any one of the 5^{th} or 6th embodiments, the metal is copper, zinc, or preferably silver, more preferably silver cations.

According to an 8^{th} embodiment, in the textile material according to any one of 5^{th} to 7^{th} embodiment, the azole-based compound is thiabendazole or a triazole-based compound.

According to a 9^{th} embodiment, in the textile material according to the 8^{th} embodiment, the triazole-based compound is propiconazole.

According to a 10^{th} embodiment, in the textile material according to any one of the preceding embodiments, the one or more antimicrobial agents are nonionic or cationic.

According to an 11^{th} embodiment, in the textile material according to any one of the preceding embodiments, the one or more antimicrobial agents are bound to the textile material either directly, in particular if the agent is a quaternary ammonium organosilane compound, polyglucosamine, or polyhexamethylene biguanide, or by means of an inorganic or organic matrix directly bound to the textile material, in particular if the agent is silver cations, or via cross linking, in particular if the agent is an azole-based compound.

According to a 12^{th} embodiment, in the textile material according to any one of the preceding embodiment, one or more of the one or more antimicrobial agents are bound to the textile material without the cyclodextrin and/or an inclusion complex, in particular without an inclusion complex of fiber-reactive cyclodextrin derivatives and antimicrobial agents.

According to a 13^{th} embodiment, in the textile material according to any one of the preceding embodiments, the one or more antimicrobial agents are not in the form of nanoparticles.

According to a 14^{th} embodiment, in the textile material according to any one of the preceding embodiments, the one or more antimicrobial agents have a particle size, in all dimensions (length, width, height), of at least 250 nanometers, preferably at least 500 nanometers, more preferably at least 750 nanometers, and most preferably at least 1,000 nanometers.

### Concentrations

According to a 15^{th} embodiment, in the textile material according to any one of the preceding embodiments, the total amount of the one or more antimicrobial agents adhered to the textile material is at most 3.0%, preferably at most 2.5%, more preferably at most 2.0%, particularly at most 1.8%, and most preferably at most about 1.4% on weight fabric of the textile material.

According to a 16^{th} embodiment, in the textile material according to any one of the preceding embodiments, the total amount of the one or more antimicrobial agents adhered to the textile material is at least 0.05%, preferably at least 0.1%, more preferably at least 0.2%, even more preferably at least 0.4%, particularly at least 0.6%, and most preferably at least about 0.9% on weight fabric of the textile material.

According to a 17^{th} embodiment, in the textile material according to any one of 5^{th} to 16^{th} embodiments, the polyhexamethylene biguanide is adhered to the textile material in an amount of at most 1.5%, preferably at most 1.0%, more preferably at most 0.8%, and most preferably at most about 0.6% on weight fabric of the textile material.

According to a 18^{th} embodiment, in the textile material according to any one of 5th to 17th embodiments, the polyhexamethylene biguanide is adhered to the textile material in an amount of at least 0.05%, preferably at least 0.1%, more preferably at least 0.2%, even more preferably at least 0.3%, and most preferably at least about 0.4% on weight fabric of the textile material.

According to a 19^{th} embodiment, in the textile material according to any one of the 5^{th} to 18^{th} embodiments, the metal is adhered to the textile material in an amount of at most 0.1%, preferably at most 0.06%, more preferably at most 0.03%, even more preferably at most 0.017%, and most preferably at most about 0.0085% on weight fabric of the textile material.

According to a 20^{th} embodiment, in the textile material according to any one of the 5^{th} to 18^{th} embodiments, the metal is adhered to the textile material in an amount of at least 0.0001%, preferably at least 0.0005%, more preferably at least 0.001%, and most preferably at least about 0.0017% on weight fabric of the textile material.

According to a 21^{st} embodiment, in the textile material according to any one of the 5^{th} to the 20^{th} embodiments, the azole-based compound is adhered to the textile material in an amount of at most 2.0%, preferably at most 1.5%, more preferably at most 1.0%, and most preferably at most about 0.8% on weight fabric of the textile material.

According to a 22^{nd} embodiment, in the textile material according to any one of the 5^{th} to 21^{st} embodiments, the azole-based compound is adhered to the textile material in an amount of at least 0.05%, preferably at least 0.1%, more preferably at least 0.2%, even more preferably at least 0.4%, and most preferably at least about 0.8% on weight fabric of the textile material.

According to a 23^{rd} embodiment, in the textile material according to any one of the 5^{th} to 22^{nd} embodiments, the polyglucosamine compound is adhered to the textile material in an amount of at most 1.5%, preferably at most 1.0%, more preferably at most 0.7%, in particular at most 0.5%, and most preferably at most about 0.3% on weight fabric of the textile material.

According to a 24^{th} embodiment, in the textile material according to any one of the 5^{th} to 23^{rd} embodiments, the polyglucosamine is adhered to the textile material in an amount of at least 0.05%, preferably at least 0.1%, more preferably at least 0.2%, and most preferably at least 0.3% on weight fabric of the textile material.

According to a 25^{th} embodiment, in the textile material according to any one of the 5^{th} to 24^{th} embodiments, the quaternary ammonium organosilane compound is adhered to the textile material in an amount of at most 0.8%, preferably at most 0.4%, more preferably at most 0.3%, in particular at most 0.2%, and most preferably at most about 0.07% on weight fabric of the textile material.

According to a 26^{th} embodiment, in the textile material according to any one of the 5^{th} to 25^{th} embodiments, the quaternary ammonium organosilane compound is adhered to the textile material in an amount of at least 0.01%, preferably at least 0.02%, more preferably at least 0.04%, and most preferably at least about 0.07% on weight fabric of the textile material.

### Efficiency

According to a 27^{th} embodiment, in the textile material according to any one of the preceding embodiments, the one or more antimicrobial agents adhered to the textile material increase the reduction value of the textile material and/or the textile material exhibits a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 measured in accordance with AATCC test method 100-2012 and/or ASTM E2149-10 by/of at least 90% (1 log), preferably at least 99% (2 log), more preferably at least 99.9% (3 log) within 10 minutes of contact time and/or of at least 99% (2 log), preferably at least 99.9% (3 log), more preferably at least 99.99% (4 log) within 1 hour of contact time and/or of at least 99.% (2 log), preferably at least 99.9% (3 log), more preferably at least 99.99% (4 log), most preferably at least 99.999% (5 log) within 24 hours of contact time.

According to a 28^{th} embodiment, in the textile material according to any one of the preceding embodiments, the textile material exhibits after 25 laundry washes a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 of at least 99%, preferably at least 99.9%, within 10 minutes on continuous reinoculations followed by dry and wet alternate abrasion cycles, preferably when tested in accordance with EPA protocol 90072PA₄ as modified.

According to a 29^{th} embodiment, in the textile material according to the 28^{th} embodiment, a laundry wash is in a laundry washing machine at 85±15 °C for 40-50 minutes, preferably using brand name non-antimicrobial, non-ionic and non-chlorine containing laundry detergent, preferably followed by a standard rinse cycle and preferably dried at 62-96 °C for 20-30 minutes.

According to a 30^{th} embodiment, in the textile material according to any one of the preceding embodiments, when sanitizing a ceramic surface by wiping a textile material over the surface, no liquid antimicrobial agent is applied to the surface and/or at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material is released from the textile material onto the surface, a reduction of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 on the surface by at least 99% (2 log), preferably at least 99.9% (3 log), more preferably at least 99.99% (4 log), even more preferably at least 99.999% (5 log), particularly at least 99.9999% (6 log), preferably evaluated after 0, 15, and/or 60 minutes post wiping can be achieved, preferably using ISO test method 18539:2004.

According to a 31^{th} embodiment, in the textile material according to the 30^{th} embodiment, the reduction can be achieved when the textile material is dry during wiping it over the surface.

According to a 32^{nd} embodiment, in the textile material according to any one of the 30^{th} or 31^{st} embodiments, the reduction can be achieved by wiping the textile material over the surface for at most 90 seconds, preferably for at most 60 seconds, more preferably for at most 30 seconds.

According to a 33^{rd} embodiment, in the textile material according to any one of the 30^{th} to 32^{nd} embodiments, the reduction can be achieved by exercising an average pressure on at least parts of the textile by a hand of the user or other wiping tool of at most 250 g/cm², preferably at most 200 g/cm², more preferably at most 150 g/cm², most preferably at most about 120 g/cm² during wiping the textile material over the surface, and/or a maximum pressure of at most 500 g/cm², preferably at most 400 g/cm², more preferably at most 300 g/cm², most preferably at most about 200 g/cm².

### Hydrophilic agents/stain release agents

### Types

According to a 34^{th} embodiment, in the textile material according to any one of the preceding embodiments, the one or more hydrophilic agents adhered to the textile material comprise at least one selected from the group consisting of carboxylic acid esters, polyester ether copolymers, starch based emulsions, fatty alcohol ethoxylates, and organosilane terpolymers, preferably fatty alcohol ethoxylates or organosilane terpolymers, most preferably organosilane terpolymers.

According to a 35^{th} embodiment, in the textile material according to any one of the preceding embodiments, the one or more stain release agents adhered to the textile material comprise at least one selected from the group consisting of fatty alcohol ethoxylates and organosilane terpolymers, preferably organosilane terpolymers.

According to a 36^{th} embodiment, in the textile material according to any one of the preceding embodiments, one or more or all of the hydrophilic agents is/are (a) stain release agent(s).

According to a 37^{th} embodiment, in the textile material according to any one of the preceding embodiments, one or more or all of the stain release agents is/are (a) hydrophilic agent(s).

### Concentrations

According to a 38^{th} embodiment, in the textile material according to any one of the preceding embodiments, the hydrophilic and/or stain release agents are adhered to the textile material in an amount of together at most 5%, preferably at most 4%, more preferably at most 3%, even more preferably at most 2%, in particular at most 1.5%, and most preferably at most about 1% on weight fabric of the textile material.

According to a 39^{th} embodiment, in the textile material according to any one of the preceding embodiments, the hydrophilic and/or stain release agents are adhered to the textile material in an amount of together at least 0.05%, preferably at least 0.1%, more preferably at least 0.2%, particularly at least 0.3%, and most preferably at least about 0.4% on weight fabric of the textile material.

According to a 40^{th} embodiment, in the textile material according to any one of the 34^{th} or 35^{th} embodiments, or any one of 36^{th} to 39^{th} embodiments when dependent from any one of the 34^{th} or 35^{th} embodiments, the organosilane terpolymers are adhered to the textile material in an amount of at least 0.1%, preferably at least 0.2%, more preferably at least 0.5%, particularly at least 0.7%, and most preferably at least about 1% on weight fabric of the textile material.

According to a 41^{st} embodiment, in the textile material according to any one of the 34^{th} or 35^{th} embodiments, or any one of 36^{th} or 40^{th} embodiments when dependent from any one of the 34^{th} or 35^{th} embodiments, the fatty alcohol ethoxylates are adhered to the textile material in an amount of at most 3%, preferably at most 2%, more preferably at most 1.5%, even more preferably at most 1%, and most preferably at most about 0.7% on weight fabric of the textile material.

### Efficiency

According to a 42^{nd} embodiment, in the textile material according to any one of the preceding embodiments, the one or more hydrophilic agents adhered to the textile material reduce the water absorbency time by at least 20%, preferably at least 40%, more preferably at least 60%, and most preferably at least 80%, preferably when measured in accordance with AATCC test method 79-2014 (Option A).

According to a 43^{rd} embodiment, in the textile material according to any one of the preceding embodiments, exhibits a water absorbency time of at most 5 seconds, preferably at most 3 seconds, more preferably at most 2 seconds, and most preferably at most 1 second, preferably when measured in accordance with AATCC test method 79-2014 (Option A).

According to a 44^{th}, in the textile material according to any one of the preceding embodiments, the one or more hydrophilic agents adhered to the textile material do not increase the water repellency of the textile material, measured in accordance with AATCC test method 22-2014.

According to a 45^{th} embodiment, in the textile material according to any one of the preceding embodiments, exhibits a water repellency rating of at most 50, preferably 0, measured in accordance with AATCC test method 22-2014.

According to a 46^{th} embodiment, in the textile material according to any one of the preceding embodiments, the one or more hydrophilic agents adhered to the textile material increase the vertical wicking rate by at least a 10%, preferably at least 20%, and most preferably at least 30%, when tested according to AATCC test method 197-2013.

According to a 47^{th} embodiment, in the textile material according to any one of the preceding embodiments, exhibits a vertical wicking rate of at least 0.15 mm/sec, preferably at least 0.18 mm/sec, more preferably at least 0.20 mm/sec, and most preferably at least 0.22 mm/sec, when tested according to AATCC test method 197-2013.

According to a 48^{th} embodiment, in the textile material according to any one of the preceding embodiments, the one or more hydrophilic agents adhered to the textile material increase the horizontal wicking rate by at least 50%, preferably 100%, more preferably 300% and most preferably 500% when tested according to AATCC test method 198-2013.

According to a 49^{th} embodiment, in the textile material according to any one of the preceding embodiments, exhibits a horizontal wicking rate of at least 15 mm²/sec, preferably at least 20 mm²/sec, more preferably at least 25 mm²/sec, and most preferably at least 30 mm²/sec, when tested according to AATCC test method 197-2013.

According to a 50^{th} embodiment, in the textile material according to any one of the preceding embodiments, the one or more stain release agents adhered to the textile material increase the stain release rating of the textile material by at least one grade, more preferably by at least two grades, particularly at least three grades, and most preferably four grades, when tested according to AATCC test method 130-2010.

According to a 51^{st} embodiment, in the textile material according to any one of the preceding embodiments, has a stain release rating, measured in accordance with AATCC test method 130-2010, of at least grade 3, preferably at least grade 4, and most preferably grade 5.

According to a 52^{nd} embodiment, in the textile material according to any one of the preceding embodiments, the one or more stain release agents adhered to the textile material increase the stain/oil repellency rating of the textile material, measured in accordance with AATCC test method 118-2013, by at most one grade, preferably do not increase the stain/oil repellency rating.

According to a 53^{rd} embodiment, in the textile material according to any one of the preceding embodiments, the textile material exhibits a stain/oil repellency rating, measured in accordance with AATCC test method 118-2013, of at most grade 1, preferably grade o.

### Wash durability

According to a 54^{th} embodiment, in the textile material according to any one of the preceding embodiments, the properties of the textile material, such as stain release capability, antimicrobial efficiency, and/or properties related to hydrophilicity are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

### Starting textile material

According to a 55^{th} embodiment, in the textile material according to any one of the preceding embodiments, the starting textile material comprises functional groups having the ability to bond one or more antimicrobial agents to the textile material.

According to a 56^{th} embodiment, in the textile material according to any one of the preceding embodiments, the starting textile material comprises hydroxyl, peptide and/or carbonyl groups, in particular hydroxyl and/or peptide.

According to a 57^{th} embodiment, in the textile material according to any one of the preceding embodiments, the starting textile material is a cellulosic textile material, a synthetic textile material, or a blend comprising a cellulosic textile material and a synthetic textile material.

According to a 58^{th} embodiment, in the textile material according to the 57^{th} embodiment, the cellulosic textile material comprises cotton, viscose, rayon, linen, hemp, ramie, jute, and combinations (blends) thereof, preferably cotton or viscose or combinations thereof.

According to a 59^{th} embodiment, in the textile material of any one according to the 57^{th} or 58^{th} embodiments, the synthetic textile material comprises one or more selected from the group consisting of polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester.

According to a 60^{th} embodiment, in the textile material according to any one of the 57^{th} to 59^{th} embodiments, the blend of cellulosic textile material and synthetic textile material comprises between 20% and 80% of cellulosic textile material, preferably between 30% and 75%, more preferably between 50% and 70%, and most preferably about 60%.

According to a 61^{st} embodiment, in the textile material according to any one of the 55^{th} to 60^{th} embodiment, the blend of cellulosic textile material and synthetic textile material comprises between 20 and 80% of synthetic textile material, preferably between 25 and 70%, more preferably between 30 and 50%, and most preferably about 40%.

According to a 62^{nd} embodiment, in the textile material according to any one of the preceding embodiments, the textile material is selected from the group consisting of woven, knitted, warp knitted, crocheted, and non-woven fabrics.

According to a 63^{rd} embodiment, in the textile material according to any one of the preceding embodiments, the textile material is a raised fabric.

According to a 64^{th} embodiment, in the textile material according to the 63^{rd} embodiment, the raised fabric is a looped fabric or has a peach finishing or a brushed finishing.

According to a 65^{th} embodiment, in the textile material according to the 64^{th} embodiment, the looped fabric is a terry fleece or polar fleece.

### Manufacturing process

A 66^{th} embodiment of the invention is a process for finishing a textile material, comprising the steps of:
- treating the textile material using an antimicrobial liquor application process like an exhaustion or padding process, wherein the liquor comprises one or more antimicrobial agents;
- preferably drying the textile material and treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

According to a 67^{th} embodiment, in process for finishing a textile material of the 66^{th} embodiment, the antimicrobial liquor application process is preferably an exhaust process, and between the step of treating the textile material using an antimicrobial liquor application process and the steps of drying the textile material and treating the textile material using a hydrophilic/stain release liquor application process, one or more process cycles are performed, comprising the steps of drying the textile material and treating the textile material using a further antimicrobial liquor application process, preferably a padding process, wherein the liquor comprises one or more antimicrobial agents.

A 68^{th} embodiment of the invention is a process for finishing a textile material, comprising the steps of:
- preferably treating the textile material using an antimicrobial liquor application process like an exhaustion or padding process, wherein the liquor comprises one or more antimicrobial agents and drying the textile material;
- treating the textile material using a hydrophilic/stain release liquor application process like an exhaustion or padding process, wherein the liquor comprises one or more hydrophilic agents and/or stain release agents; and
- subjecting the treated textile material to a heat treatment.

### Liquor

According to a 69^{th} embodiment, in the process of any one of the 66^{th} to 68^{th} embodiments, the liquor of an antimicrobial and/or hydrophilic/stain release liquor application process has a pH-value of at most 6.9, preferably at most 6.5, more preferably at most 6.3, in particular at most 6.0, and most preferably at most about 5.5, and a pH-value of at least 3.0, preferably at least 3.5, more preferably at least 4.0, even more preferably at least 4.5, in particular at least 5.0, and most preferably at least about 5.5.

According to a 70^{th} embodiment, in the process of any one of the 66^{th} or 69^{th} embodiments, the amount of stain release and/or hydrophilic agents in the liquor of an antimicrobial liquor application process is together at most 10 gpl, preferably at most 5 gpl, more preferably at most 2 gpl, even more preferably at most 1 gpl, and most preferably o.

According to a 71th embodiment, in the process of any one of the 66^{th} or 70^{th} embodiment, in an antimicrobial and/or the hydrophilic/stain release liquor application process, the agents and preferably all other components in the liquor are dissolved in the liquor, in particular are not dispersed in the liquor, and/or the liquor is substantially free of solids.

According to a 72^{nd} embodiment, in the process of any one of 1^{st} to 71^{st} embodiments, the liquor of an antimicrobial and/or hydrophilic/stain release liquor application process contains a solvent, preferably water, and the agents and the solvent form a homogenous mixture, and in particular do not form a slurry or dispersion.

According to a 73^{rd} embodiment, in the process of any one of the 66^{th} to 72^{nd} embodiments, the value of dynamic viscosity of the liquor of an antimicrobial and/or hydrophilic/stain release liquor application process at 20 °C and/or 80 °C, in centipoise (cP), is at most 20% higher than the dynamic viscosity of water at 20 °C and/or 80 °C, respectively, preferably at most 10%, more preferably at most 5%, particularly at most 2%, and most preferably at most about 0%.

According to a 74^{th} embodiment, in the process of any one of the 66^{th} to 73^{rd} embodiments, the amount of latex in the liquor of an antimicrobial and/or the hydrophilic/stain release liquor application process is at most 10 gpl, preferably at most 5 gpl, more preferably at most 2 gpl, even more preferably at most 1 gpl, and most preferably o.

According to a 75^{th} embodiment, in the process of any one of the 66^{th} to 73^{rd} embodiments, the amount of cyclodextrin and/or inclusion complexes, in particular inclusion complexes of fiber-reactive cyclodextrin derivatives and antimicrobial agents in the liquor of an antimicrobial and/or the hydrophilic/stain release liquor application process is at most 10 gpl, preferably at most 5 gpl, more preferably at most 2 gpl, even more preferably at most 1 gpl, and most preferably o.

According to a 76^{th} embodiment, in the process of any one of the 66^{th} to 75^{th} embodiment, the amount of dye in the liquor of an antimicrobial and/or main process cycle is at most 10 gpl, preferably at most 5 gpl, more preferably at most 2 gpl, even more preferably at most 1 gpl, and most preferably o.

### Drying and curing

According to a 77^{th} embodiment, in the process of any one of the 66^{th} to 76^{th} embodiments, the drying is conducted at least partially at an ambient temperature of at least 70 °C, preferably at least 100 °C, more preferably at least 110 °C, most preferably at least about 120 °C.

According to a 78^{th} embodiment, in the process of any one of the 66^{th} to 77^{th} embodiments, the drying is conducted at an ambient temperature of at most 160 °C, preferably of at most 140 °C, more preferably of at most 130 °C, and most preferably of at most about 120 °C.

According to a 79^{th} embodiment, in the process of any one of the 66^{th} to 78^{th} embodiments, the heat treatment comprises drying conducted at least partially at an ambient temperature of at least 60 °C, in particular at least 100 °C, preferably at least 110 °C, more preferably at least 120 °C.

According to an 80^{th} embodiment, in the process of any one of the 66^{th} to 79^{th} embodiments, the heat treatment comprises curing of the textile material, wherein the curing is conducted at least partially at a curing ambient temperature of at least 140 °C, preferably at least 160 °C, more preferably at least 170 °C, particularly at least 175 °C, and most preferably at least about 180 °C.

According to an 81^{st} embodiment, in the process of any one of the 66^{th} to 80^{th} embodiments, the heat treatment is conducted at an ambient temperature of at most 200 °C, preferably at most 190 °C, more preferably at most 185 °C, and most preferably at most about 180 °C.

According to an 82^{nd} embodiment, in the process of any one of the 80^{th} to 81^{st} embodiments, curing takes place at the curing temperature as defined in the 80^{th} embodiment over a period of at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

According to an 83^{rd} embodiment, in the process of any one of the 80^{th} to 82^{nd} embodiments, the textile material is a fabric of at least 350 grams per m² and curing takes place at the curing temperature as defined in the 66^{th} embodiment over a period of at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

According to an 84^{th} embodiment, in the process of any one of the 80^{th} to 83^{rd} embodiments, the textile material is a fabric of at least 500 grams per m² and curing takes place at the curing temperature as defined in the 66^{th} embodiment over a period of at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds.

According to an 84^{th} embodiment, in the process of any one of the 80^{th} to 84^{th} embodiments, curing immediately follows drying of the textile material without the textile material substantially cooling down between drying of the textile material and curing.

According to an 85^{th} embodiment, in the process of the 84^{th} embodiment, the textile material is a fabric and drying of the textile material and curing are performed over a period of together at least 45 seconds, preferably at least 50 seconds, more preferably at least 55 seconds, most preferably at least about 60 seconds, per 100 grams of fabric weight per square meter.

According to an 86^{th} embodiment, in the process of any one of the 84^{th} or 85^{th} embodiment, the textile material is a fabric and drying of the textile material and curing are performed over a period of together at most 75 seconds, preferably at most 70 seconds, more preferably at most 65 seconds, most preferably at most about 60 seconds, per 100 grams of fabric weight per square meter.

### Exhaustion and padding parameters:

According to an 87^{th} embodiment, in the process of any one of the 66^{th} to 86^{th} embodiments, where an antimicrobial and/or hydrophilic/stain release liquor application process is an exhaustion process, the liquor has a temperature of at least 45 °C, preferably of at least 50 °C, more preferably of at least 60 °C, particularly of at least 70 °C, and most preferably of at least about 80 °C.

According to an 88^{th} embodiment, in the process of any one of the 66^{th} to 79^{th} embodiments, where the antimicrobial and/or hydrophilic/stain release liquor application process is an exhaustion process, the liquor has a temperature below boiling temperature, preferably of at most 95 °C, more preferably of at most 90 °C, particularly of at most 85 °C, and most preferably of at most about 80 °C.

According to an 89^{th} embodiment, in the the process of any one of the 66^{th} to 88^{th} embodiments, the exhaustion process time is at most 90 min, preferably at most 80 min, more preferably at most 70 min, and most preferably at most about 60 min.

According to a 90^{th} embodiment, in the process of any one of the 66^{th} to 89^{th} embodiments, he exhaustion process time is at least 45 min, preferably at least 50 min, more preferably at least 55 min, and most preferably at least about 60 min.

According to a 91^{st} embodiment, in the process of any one of the 66^{th} to 90^{th} embodiment, in a padding process of an antimicrobial and/or hydrophilic/stain release liquor application process, the liquor pickup rate is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%

According to a 92^{nd} embodiment, in the process of any one of the 66^{th} to 91^{st} embodiments, in a padding process of an antimicrobial and/or hydrophilic/stain release liquor application process, the textile material passes through a multiple trough padding mangle, or a continuous dyeing or bleaching range.

### Agents:

According to a 93^{rd} embodiment, in the process of any one of the 66^{th} to 92^{nd} embodiments, the one or more antimicrobial agents are selected and/or applied as defined in any one of the 5^{th} to 14^{th} embodiments.

According to a 94^{th} embodiment, in the process of any one of the 66^{th} to 93^{rd} embodiments, the antimicrobial agents are adhered to the textile material with a total weight as defined in the 15^{th} or 16^{th} embodiments, and/or an individual weight as defined for the respective antimicrobial agents in any one of the 17^{th} to 26^{th} embodiments.

According to a 95^{th} embodiment, in the process of any one of the 66^{th} to 94^{th} embodiments, the one or more hydrophilic agents are selected and/or applied as defined in the 34^{th} embodiment.

According to a 96^{th} embodiment, in the process of any one of the 66^{th} to 95^{th} embodiments, the one or more stain release agents are selected and/or applied as defined in the 35^{th} embodiment.

According to a 97^{th} embodiment, in the process of any one of the 66^{th} to 96^{th} embodiments, the hydrophilic and/or stain release agents are adhered to the textile material with a total weight as defined in any one of the 38^{th} to 41^{st} embodiments.

### Starting textile material:

According to a 98^{th} embodiment, in the process of any one of the 66^{th} to 97^{th} embodiments, the (starting) textile material is a material as defined in any one of the 55^{th} to 65^{th} embodiments.

### Other references between process and product embodiments:

According to a 99^{th} embodiment, in the process of any one of embothe 66^{th} to 98^{th} embodiments, the textile material obtained by the process is a textile material according to any one of the 1^{st} to 65^{th} embodiments.

A 100^{th} embodiment is a textile material obtainable according to the process of any one of the 66^{th} to 99^{th} embodiments.

### Some preferred embodiments

According to a 101^{st} embodiment, in the textile material according to any one of the 5^{th} to 26^{th} embodiments, or 100^{th} embodiment, the textile material preferably comprises or consists of a cellulosic textile material, and wherein
- the azole-based compound is adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- the metal is adhered to the textile material in an amount of at least 0.0002%, preferably at least 0.0005%, more preferably at least 0.001%, particularly at least 0.0013%, most preferably at least about 0.0017%, and/or in an amount of at most 0.02%, preferably at most 0.01%, more preferably at most 0.005%, particularly at most 0.003%, most preferably at most about 0.0017% on weight fabric of the textile material, and/or
- the PHMB is adhered to the textile material in an amount of at least 0.05%, preferably at least 0.1%, more preferably at least 0.2%, particularly at least 0.4%, and most preferably at least about 0.6%, and/or in an amount of at most 2.0%, preferably at most 1.5%, more preferably at most 1.0%, particularly at most 0.8%, most preferably at most about 0.6% on weight fabric of the textile material.

According to a 102^{nd} embodiment, in the textile material according to any one of the 5^{th} to 26^{th} embodiments, or the 100^{th} embodiment, the textile material preferably comprises or consists of a blend of cellulosic textile material and synthetic textile material, and wherein
- the azole-based compound is adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- the metal is adhered to the textile material in an amount of at least 0.001%, preferably at least 0.002%, more preferably at least 0.004%, particularly at least 0.006%, most preferably at least about 0.0085%, and/or in an amount of at most 0.1%, preferably at most 0.05%, more preferably at most 0.03%, particularly at most 0.017%, most preferably at most about 0.0085% on weight fabric of the textile material, and/or
- the PHMB is adhered to the textile material in an amount of at least 0.05%, preferably at least 0.1%, more preferably at least 0.2%, particularly at least 0.3%, and most preferably at least about 0.4%, and/or in an amount of at most 1.5%, preferably at most 1.0%, more preferably at most 0.8%, particularly at most 0.6%, most preferably at most about 0.4% on weight fabric of the textile material.

According to a 103^{rd} embodiment, in the textile material according to any one of the 5^{th} to 26^{th} embodiments, or the 100^{th} embodiment, the textile material preferably comprises substantially no cellulosic textile material, and
- the azole-based compound is adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- the metal is adhered to the textile material in an amount of at least 0.001%, preferably at least 0.002%, more preferably at least 0.004%, particularly at least 0.006%, most preferably at least about 0.0085%, and/or in an amount of at most 0.1%, preferably at most 0.05%, more preferably at most 0.03%, particularly at most 0.017%, most preferably at most about 0.0085% on weight fabric of the textile material, and/or
- the quaternary ammonium organosilane compound is adhered to the textile material in an amount of at least 0.005%, preferably at least 0.01%, more preferably at least 0.02%, particularly at least 0.04%, and most preferably at least about 0.07%, and/or in an amount of at most 0.5%, preferably at most 0.3%, more preferably at most 0.2%, particularly at most 0.15%, most preferably at most about 0.07% on weight fabric of the textile material.

### Use as a surface sanitizer

A 104^{th} embodiment of the invention is a method of sanitizing a surface by wiping a textile material according to any of the 1^{st} to 62^{nd} embodiments or any one of the 100^{th} to 103^{rd} embodiments over the surface.

According to a 105^{th} embodiment, in the method of sanitizing a surface according to the 104^{th} embodiment, sanitizing means a reduction of at least certain types of living bacteria such as *Escherichia coli* ATCC 25922 and/or *Staphylococcus aureus* ATCC 6538 and/or *Pseudomonas aeroginosa* ATCC 15442 and/or *Salmonella enterica* ATCC 10708, preferably most types of living bacteria, more preferably all types of living bacteria, and/or a reduction of *Candida albicans* ATCC 10231 and/or *Aspergillus niger* 16404 on the surface by at least 99% (2 log), preferably at least 99.9% (3 log), more preferably at least 99.99% (4 log), even more preferably at least 99.999% (5 log), particularly at least 99.9999% (6 log), preferably evaluated after 0, 15, and/or 60 minutes post wiping.

According to a 106^{th} embodiment, in the method of sanitizing a surface of any one of the 104^{th} or 105^{th} embodiments, at least 50%, preferably at least 90% (1 log), more preferably at least 99% (2 log), even more preferably at least 99.9% (3 log), particularly at least 99.99% (4 log) or 99.999% (5 log), and most preferably at least 99.9999% (6 log) of one or more or preferably all types of living bacteria and/or other microbes picked up by the textile material are killed, preferably on the textile material, and more preferably by the antimicrobial agents adhered to the textile material, preferably within 60 minutes, more preferably within 30 minutes, even more preferably within 15 minutes post wiping.

According to a 107^{th} embodiment, in the method of sanitizing a surface of any one of the 104^{th} to 106^{th} embodiments, no liquid antimicrobial agent is applied to the surface and/or wherein at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material is released from the textile material onto the surface.

According to a 108^{th} embodiment, in the method of sanitizing a surface of any one of the 104^{th} to 107^{th} embodiments, liquid antimicrobial agent is applied to the surface and/or wherein more than 1% of an antimicrobial agent, in particular of a liquid antimicrobial agent, adhered to, contained in or held by the textile material is released from the textile material onto the surface, but the sanitizing would be achieved even if no liquid antimicrobial agent was used and/or if at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material was released from the textile material onto the surface.

According to a 109^{th} embodiment, in the method of sanitizing a surface of any one of the 104^{th} to 108^{th} embodiments, the textile material and/or the surface is wetted and/or a cleaning agent is applied to the textile material and/or the surface prior to wiping the textile material over the surface.

According to a 110^{th} embodiment, in the method of sanitizing a surface of any one of the 104^{th} to 107^{th} embodiments, the textile material is dry during wiping it over the surface.

According to a 111^{th} embodiment, in the method of sanitizing a surface of any one of the 104^{th} to 110^{th} embodiments, wiping the textile material over the surface is performed at least once, for at least 5 seconds, for at least 10 seconds, for at least 30 seconds, for at least 60 seconds, or for at least 90 seconds.

According to a 112^{th} embodiment, in the method of sanitizing a surface of any one of the 104^{th} to 111^{th} embodiments, wiping the textile material over the surface is performed for at most 90 seconds, preferably for at most 60 seconds, more preferably for at most 30 seconds, particularly at most 10 seconds, and most preferably only once.

According to a 113^{th} embodiment, in the method of sanitizing a surface of any one of the 104^{th} to 112^{th} embodiments, during wiping the textile material over the surface, an average pressure of at least 5 g/cm², preferably at least 10 g/cm², more preferably at least 25 g/cm², even more preferably at least 50 g/cm², particularly at least about 120 g/cm² is exercised on at least parts of the textile by a hand of the user or other wiping tool, and/or a maximum pressure of at most 500 g/cm², preferably at most 400 g/cm², more preferably at most 300 g/cm², most preferably at most about 200 g/cm².

According to a 114^{th} embodiment, in the method of sanitizing a surface of any one of the 104^{th} to 113^{th} embodiments, during wiping the textile material over the surface, an average pressure of at most 600, preferably at most 500 g/cm², more preferably at most 400 g/cm², particularly at most 300 g/cm², and most preferably at most 200 g/cm² is exercised on at least parts of the textile by a hand of the user or other wiping tool.

According to a 115^{th} embodiment, in the method of sanitizing a surface of any one of the 104^{th} to 114^{th} embodiments, the surface is living human or animal skin, preferably skin of the human hand or face.

According to a 116^{th} embodiment, in the method of sanitizing a surface of any one of the 104^{th} to 115^{th} embodiments, the surface is a floor, a kitchen top, a table, the surface of a medical device, a surface of furniture in a doctor's office or in a hospital, in particular in an operating theater or emergency room, the surface of a door handle, or the surface of the housing of an electrical consumer product.

According to a 117^{th} embodiment, in the method of sanitizing a surface of any one of the 104^{th} to 116^{th} embodiments, the surface is dried by the wiping of the textile material over the surface.

According to a 118^{th} embodiment, in the method of sanitizing a surface of any one of the 104^{th} to 117^{th} embodiments,
aqueous, oily, or other liquids, in particular body liquids like sweat or blood, food rests, dust, stains, or other dirt or dirt particles is/are removed from the surface and/or the surface is cleaned of such materials, by the wiping of the textile material over the surface.

A 119^{th} embodiment of the invention is a handkerchief, towel, in particular face, kitchen, hospital, or baby towel, a floor mob, or a duster, in particular table duster, consisting of or comprising the textile material according to any of the 1^{st} to 62^{nd} embodiments or the 100^{th} embodiment.

### DETAILED DESCRIPTION OF PREFERRED EMBODIMENTS

In the following, preferred embodiments of the invention are described with reference to the figures, in which:
- Fig. 1: is a flowchart illustrating a process of finishing a textile material according to embodiments of the invention;
- Fig. 2: is a flowchart illustrating a process of finishing a textile material according to a preferred embodiment of the invention;
- Fig. 3A and 3B: specify a cotton and a polyester textile material according to working examples of the invention;
- Fig. 4AA to 4BC: specify textile materials according to experimental examples of the invention, wherein one single antimicrobial agent was applied;
- Fig. 5AA to 5BC: specify textile materials according to experimental examples of the invention, wherein two antimicrobial agents were applied;
- Fig. 6A and 6B: specify textile materials according to experimental examples of the invention, wherein three antimicrobial agents were applied;
- Fig. 7A and 7B: specify textile materials according to experimental examples of the invention, wherein only antimicrobial agents were applied;
- Fig. 8AA to 8BC: specify textile materials according to experimental examples of the invention, wherein antimicrobial agents and Hydrosil were applied;
- Fig. 9AA to 9BC: specify textile materials according to experimental examples of the invention, wherein antimicrobial agents and Permalose were applied;
- Fig. 10AA to 10BC: specify textile materials according to experimental examples of the invention, wherein different exhaust times were applied;
- Fig. 11AA to 11BD: specify textile materials according to experimental examples of the invention, wherein different curing temperatures were applied;
- Fig. 12: specifies a textile material according to an experimental example of the invention, wherein the antimicrobial agents were applied in a padding process;
- Fig. 13A to 13C: specify textile materials according to experimental examples of the invention, wherein a non-raised/peached/looped starting textile material was used.

### Antimicrobial agents

### Preferred antimicrobial agents

A great variety of antimicrobial agents can be fixed to a textile by using the process of the invention described below. The antimicrobial agents are preferably non-ionic or cationic, but not anionic. The inventors found that anionic compounds do not bind well to textiles and can easily be removed, e.g. by salts. Cationic (acid) agents are believed to attack the textile and therefore attach to it. Nanoparticles or antimicrobials in the form of nanoparticles are not preferred. Rather, the one or more antimicrobial agents have a preferred particle size, in all dimensions (length, width, height), of at least 250 nanometers, preferably at least 500 nanometers, more preferably at least 750 nanometers, and most preferably at least 1,000 nanometers.

According to the preferred embodiments of the invention, an antimicrobial agent is selected from quaternary ammonium organosilane compounds, metal, polyglucosamine (chitosan), azole-based compounds, and polyhexamethylene biguanide (PHMB).

As will be described for the preferred antimicrobial agents in detail below, the antimicrobial agents are bound to the textile material preferably either directly, in particular if the agent is a quaternary ammonium organosilane compound, polyglucosamine, a silver, copper, or zinc cation, which can be trapped in an inorganic or organic matrix, or PHMB, or via cross linking, in particular if the agent is an azole-based compound. The use of cyclodextrin and/or an inclusion complex, in particular an inclusion complex of fiber-reactive cyclodextrin derivatives and antimicrobial agents is not preferred for binding the antimicrobial agents, in particular because cyclodextrin is prohibitively expensive for most applications.

Suitable quaternary ammonium organosilane compounds have the formula 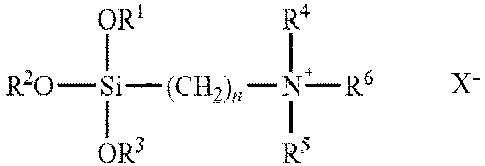 wherein the radicals have, independently of each other, the following meanings: R¹, R², and R³ are a C₁-C₁₂-alkyl group, in particular a C₁-C₆-alkyl group, preferably a methyl group; R4, and R⁵ are a C₁-C₁₈-alkyl group, a C₁-C₁₈-hydroxyalkyl group, a C₃-C₇-cycloalkyl group, a phenyl group, or a C₇-C₁₀-aralkyl group, in particular a C₁-C₁₈-alkyl group, preferably a methyl group; R⁶ is a C₁-C₁₈-alkyl group, in particular a C₈-C₁₈-alkyl group; X⁻ is the counterion and an anion, for example, chloride, bromide, fluoride, iodide, acetate, or a sulfonate group, preferably X⁻ is chloride or bromide; and n is an integer of 1 to 6, in particular an integer of 1 to 4, preferably 3. The term "alkyl group" as used herein means a branched or unbranched alkyl group.

Quaternary ammonium organosilane compounds are known in the art and commercially available. Such compounds possess specific functional groups which enable their bonding to functional groups of the textile material. Under the reaction conditions disclosed herein, the quaternary ammonium organosilane compounds are bonded to the textile material via a covalent bond between the organosilane moiety and functional groups of the textile. Furthermore, organosilane moieties polymerize with each other, resulting in -O-Si-O- bonds. A possible reaction mechanism of the ammonium organosilane with a textile material having hydroxyl groups is shown hereinafter.

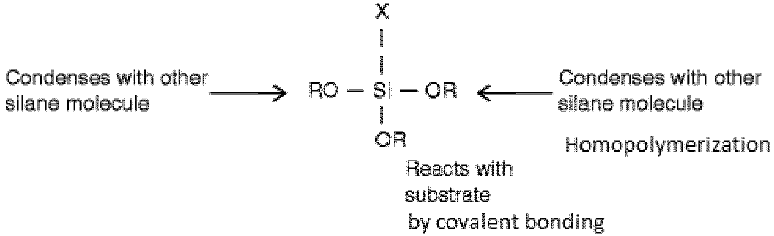

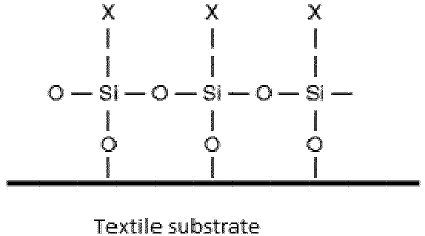

A possible reaction mechanism of the ammonium organosilane with silk having peptide groups (-CO-NH-) is shown hereinafter.

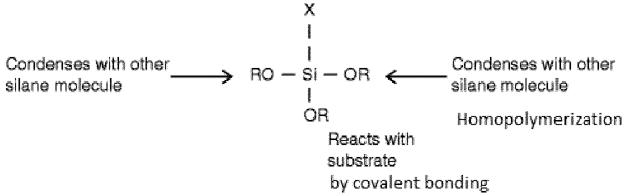

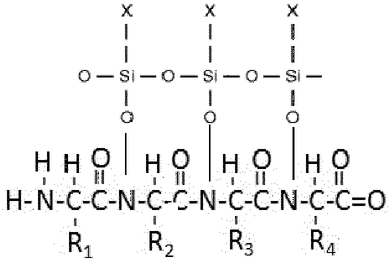

The quaternary ammonium organosilane compound can comprise dimethyloctadecyl[3-(trimethoxysilyl)propyl] ammonium chloride or dimethyltetradecyl[3-(trimethoxysilyl)propyl] ammonium chloride, most preferably dimethyloctadecyl[3-(trimethoxysilyl)propyl]ammonium chloride. The structure of dimethyloctadecyl[3-(trimethoxysilyl)propyl]ammonium is as follows (shown without counterion), wherein further the function of the silane moiety and the ammonium moiety are indicated:

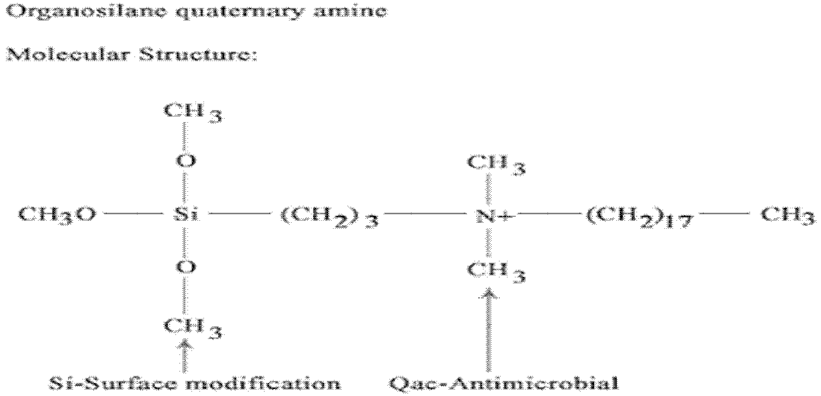

Dimethyloctadecyl[3-(trimethoxysilyl)propyl] ammonium chloride is available on the market, e.g. in AEM 5772 (manufactured by Aegis, USA). Dimethyltetradecyl[3-(trimethoxysilyl)propyl] ammonium chloride is available on the market, e.g. in Sanitized T 99-19 (manufactured by Sanitized AG, Switzerland). Other suitable ammonium silane compounds are described, e.g., in patent applications US 2011/0271873 A1 and US 2006/0193816 A1, and in US patent 8,906,115.

The metal may be copper, zinc, or preferably silver, more preferably silver cations. Tests made by the inventor, whose results are not reproduced herein, showed that copper nitrate and zinc nitrate have similar antimicrobial properties as silver cations, and they can be adhered to textile materials in similar ways. However, the antimicrobial performance of silver is considerably higher than that of zinc, and even much higher than that of copper. Furthermore, treating textile materials with copper or zinc tends to lead more easily to a change of the shade of textile than a treatment with silver.

In particular embodiments, the silver cations are trapped in an inorganic or organic matrix. Preferably, the inorganic matrix is an aluminosilicate. Preferably, the organic matrix is a polymeric matrix. Such silver-containing microbial agents are known in the art and available on the market.

A silver cation in form of its acrylate salt is shown hereinafter.

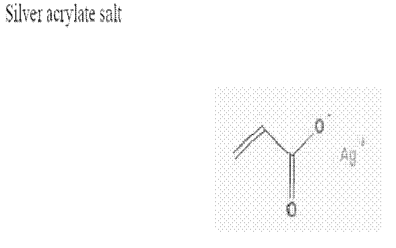

In an exemplary embodiment of the invention, the aluminosilicate is a sodium-poly(sialate-disiloxo) compound. Examples of an aluminosilicate and sialate structures as well as how bonding to a textile material can occur under the reaction conditions disclosed herein, are shown hereinafter.

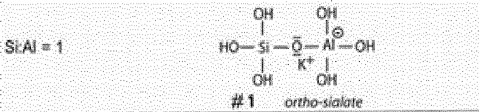

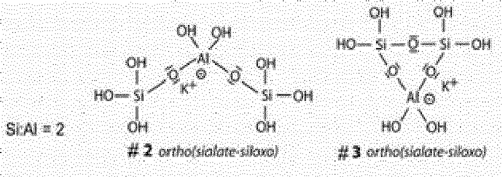

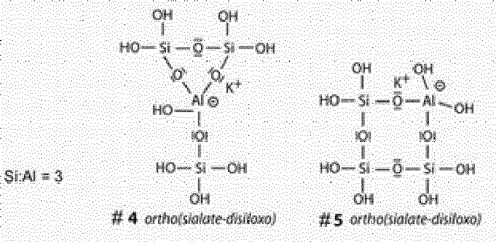

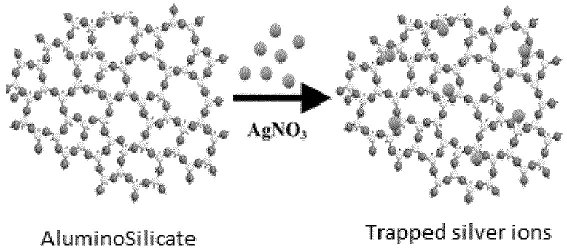

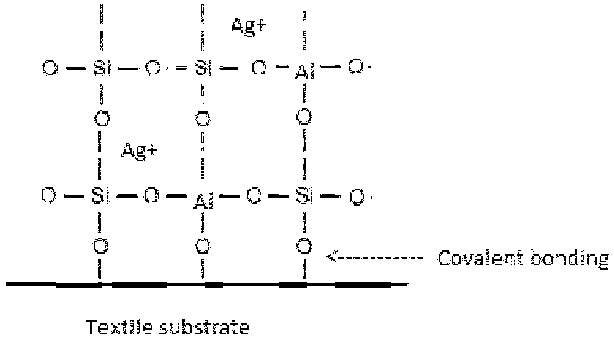

In an exemplary embodiment of the invention, the polymeric matrix into which silver cations are trapped is an acrylic polymer. Such silver-containing agents are known in the art and available on the market, e.g. in SilvaDur AQ Antimicrobial of Rohm and Haas, Switzerland, which contains acrylic polymer(s), silver nitrate, nitric acid and water. In another exemplary embodiment of the invention, the silver cations are trapped in a polymeric matrix. Such silver-containing agents are known in the art and available on the market, e.g. in SILVADUR™ 930 Antimicrobial of Dow Chemical Company, USA, which contains polymer(s), silver cations, ammonia, ethanol and water.

Polyglucosamine (chitosan) has a structure as shown hereinafter, wherein n indicates the number of monomer units as known in the art:

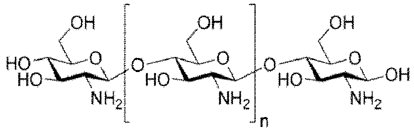

Under the reaction conditions disclosed herein, chitosan can react with -NH groups of silk resulting in covalent bonds as shown below.

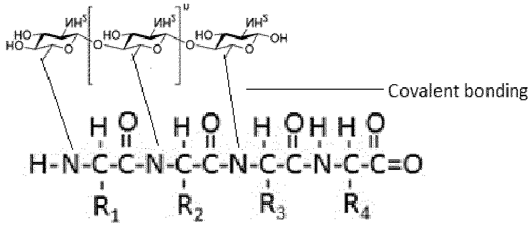

Under the reaction conditions disclosed herein, chitosan can react with functional groups of cellulosic materials resulting in covalent bonds as shown below.

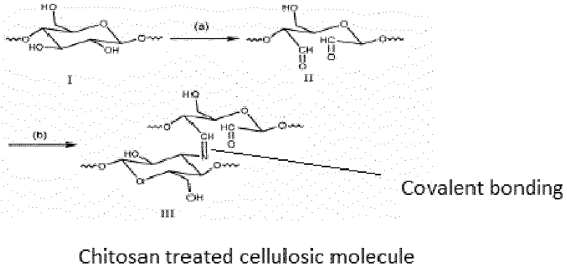

Chitosan is known in the art and commercially available, e.g. in Goyenchem-102 of Go Yen Chemical, Taiwan. It is particularly effective for killing viruses. It cannot easily be bound to synthetic textile materials in a wash-durable or even substantially non-leaching manner.

Polyhexamethylene biguanide (PHMB) has a structure as shown hereinafter, wherein n indicates the number of monomer units as known in the art.

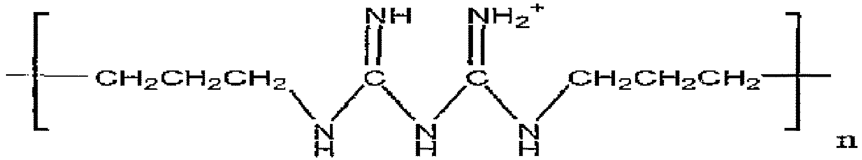

Under the reaction conditions disclosed herein, polyhexamethylene biguanide can react with hydroxyl groups of cellulose to form covalent bonds as shown hereinafter.

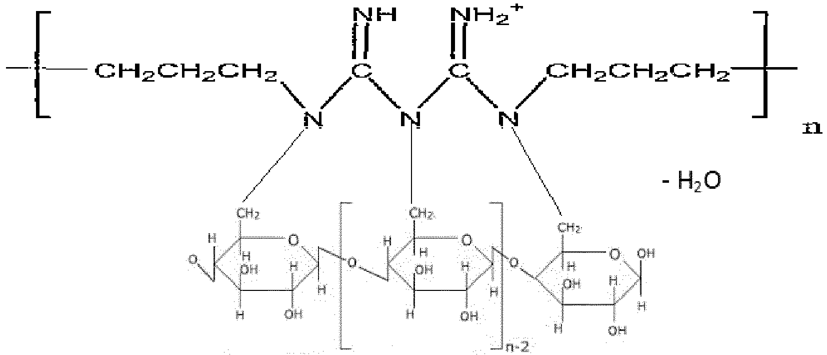

Under the reaction conditions disclosed herein, PHMB can react with carbonyl groups of silk fiber to form covalent bonds as shown hereinafter.

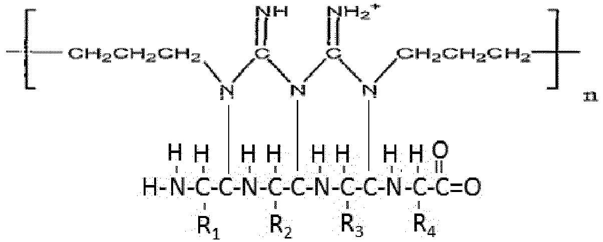

PHMB is known in the art and commercially available, e.g. in Texguard 20 of Swissol Specialties Pvt. Ltd, India. It cannot easily be bound to synthetic textile materials in a wash-durable or even substantially non-leaching manner.

The azole-based compound can be, e.g., thiabendazole, carbendazim or preferably a triazole-based compound. Tests made by the inventor, whose results are not reproduced herein, showed that thiabendazole has similar antimicrobial properties as the triazole-based compounds, and it can be adhered to textile materials in similar ways. However, the antimicrobial performance of triazole-based compounds is considerably higher than that of thiabendazole.

The triazole-based compound is preferably propiconazole. Propiconazole has a structure as shown hereinafter.

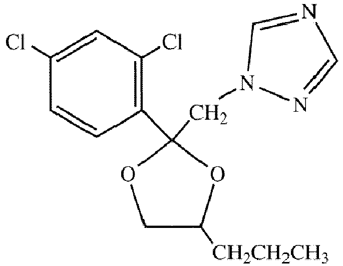

Propiconazole is known in the art and commercially available, e.g. in Biogard PPZ 250 of Beyond Surface Technologies, Switzerland. Propiconazole can be bound to the textile material using a crosslinking agent, in particular a preferably blocked isocyanate compound, which results in urethane bonds, or an acrylate based-product. When using propiconazole, it is preferred to use a crosslinking agent in the liquor, in particular an exhaust liquor. It is even more preferred that the formulation of propiconazole contains the cross linking agent or the cross-linking agent is part of the propiconazole formulation. Furthermore, it is preferred to use propiconazole together with an emulsifier. Propiconazole is particularly effective for killing fungi.

### Combinations of several antimicrobial agents

In preferred embodiments of the invention, at least two, at least three, at least four, or all five of the antimicrobial agents selected from the above-mentioned group consisting of quaternary ammonium organosilane compounds, metal, chitosan, azole-based compounds, and PHMB are adhered to a textile material.

The use of a combination of several antimicrobial agents has the following advantages over the use of a single agent:
First of all, different agents have different antimicrobial effects. Some may work better against bacteria, others against virus, and again other against fungi. Adding a variety of agents increases the spectrum of microbes which can be killed when they come in contact with the antimicrobial textile.

Secondly, the use of a variety of agents can lead to significantly higher killing rates, even for the same organism. It is believed that the higher killing rates are due to synergistic effects between the different agents. The different agents may work synergistically together due to their different killing mechanisms.

Thirdly, the use of different agents may allow binding a higher total amount of agents to the textile. For each of the different agents, there is an inherent limit on the quantity of the agent that can be adhered to the textile in a non-leaching or substantially non-leaching manner. However, even if a textile material has been saturated with and for one agent, there may still be "space" for another agent.

Forth, the use of several agents allows reducing the leaching rates per agent. It is less the total amount of leached substances that will determine the threat to health and environment, but rather the amount per substance. Thus, although the total amount of leached substances may be the same, the leaching values per agent are lower, which is highly beneficial.

Fifth, inherent undesired effects of a substance can be reduced or even counterbalanced by the use of several agents. For example, organosilane is hydrophobic by nature, which is an undesired property for many applications of textiles. For such applications, the concentration of organosilane should be kept at a minimum.

Sixth, some of the preferred agents of the present invention are more expensive than others, e.g. silver cations and chitosan. Reducing the concentrations of these agents and complementing them by other agents allows achieving the antimicrobial performance at substantially lower costs.

These advantages could be shown by the inventor in a series of experiments, some of which are presented in International patent application PCT/EP2016/054245 by the same applicant, and some of which will be presented below.

It is one merit of the invention to have recognized the advantages of using several antimicrobial agents in combination. It is another merit of the invention to have identified several highly effective antimicrobial agents which can be bound to a textile material together, which are relatively inexpensive, and low in toxicity. It is a further merit of the invention to have identified a process, which will be described below, by which many different agents can be applied to a textile material in one and the same liquor application process, be it in one or more application cycles, in a wash-durable or even substantially non-leaching manner. According to the preferred embodiments, wash-durable means that any agents that achieve a property of the textile material are essentially permanently adhered to a textile material, and the property is present even after at least 25 laundry washes in a laundry washing machine at 85±15 °C for 10-15 minutes using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

In some embodiments of the invention, at least one, preferably at least two, more preferably at least three, and most preferably all four selected from the group consisting of metal, azole-based compounds, chitosan, and PHMB are adhered to a textile material. This is the same group as the group of five mentioned above but without quaternary ammonium organosilane. Such a combination is particularly suitable where hydrophilicity is an important property of the treated textile because organosilane renders the textile mildly hydrophobic.

In some embodiments of the invention, at least one, preferably at least two, more preferably at least three, and most preferably all four selected from the group consisting of metal, azole-based compounds, quaternary ammonium organosilane compounds, and PHMB are adhered to a textile material. This is the same group as the group of five mentioned above but without chitosan. Such a combination is particularly suitable where manufacturing costs are an important issue because chitosan is relatively expensive.

In some embodiments of the invention, at least one, preferably at least two, more preferably all three selected from the group consisting of metal, azole-based compounds, and PHMB are adhered to a textile material. This is the same group as the group of five mentioned above but without chitosan and organosilane. Such a combination is particularly suitable where hydrophilicity is an important property of the treated textile and manufacturing costs are an important issue.

In some embodiments of the invention, at least one, preferably at least two, more preferably all three selected from the group consisting of metal, azole-based compounds, and quaternary ammonium organosilane compounds are adhered to a textile material. This is the same group as the group of five mentioned above but without chitosan and PHMB. Such a combination is particularly suitable where the starting textile is substantially made of a synthetic material and comprises no significant amounts of cellulosic material because chitosan and PHMB cannot easily be bound to synthetic textile materials, in particular not in a wash-durable or even substantially non-leaching manner.

In some embodiments of the invention, at azole-based compounds and at least one, preferably at least two, or all least three, selected from the group consisting of metal, PHMB and quaternary ammonium organosilane compounds are adhered to a textile material. The azole-based compounds, in particular propiconazole, are highly efficient against fungi, whereas the other three agents are highly efficient against bacteria. Therefore, azole-based compounds and at least one of the other three agents complement each other well, for manufacturing textiles that are effective against both bacteria and fungi.

### Amounts of antimicrobial agents adhered to the textile material

The ideal percentage of the chemicals present on the textile material has been elaborated in an extensive research and development effort and is in part based on the work discussed in International patent application PCT/EP2016/054245 by the same applicant.

Preferably, the total amount of the one or more antimicrobial agents adhered to the textile material is at most 3.0%, preferably at most 2.5%, more preferably at most 2.0%, particularly at most 1.8%, and most preferably at most about 1.4% on weight fabric of the textile material. These amounts represent the maximum amount of antimicrobial agents the textile can take in, and if higher amounts are applied, leaching of the agents increases significantly. Furthermore, the total amount of the one or more antimicrobial agents adhered to the textile material is preferably at least 0.05%, preferably at least 0.1%, more preferably at least 0.2%, even more preferably at least 0.4%, particularly at least 0.6%, and most preferably at least about 0.9% on weight fabric of the textile material, to ensure high antimicrobial efficiency.

In preferred embodiments, PHMB is adhered to the textile material in an amount of at most 1.5%, preferably at most 1.0%, more preferably at most 0.8%, and most preferably at most about 0.6% on weight fabric of the textile material. Furthermore, the PHMB is adhered to the textile material in an amount of preferably at least 0.05%, more preferably at least 0.1%, even more preferably at least 0.2%, in particular at least 0.3%, and most preferably at least about 0.4% on weight fabric of the textile material.

In preferred embodiments, metal is adhered to the textile material in an amount of at most 0.1%, preferably at most 0.06%, more preferably at most 0.03%, even more preferably at most 0.017%, and most preferably at most about 0.0085% on weight fabric of the textile material. Furthermore, the metal is adhered to the textile material in an amount of preferably at least 0.0001%, more preferably at least 0.0005%, even more preferably at least 0.001%, and most preferably at least about 0.0017% on weight fabric of the textile material.

In preferred embodiments, azole-based compounds are adhered to the textile material in an amount of at most 2.0%, preferably at most 1.5%, more preferably at most 1.0%, and most preferably at most about 0.8% on weight fabric of the textile material. Furthermore, the azole-based compounds are adhered to the textile material in an amount of preferably at least 0.05%, more preferably at least 0.1%, even more preferably at least 0.2%, in particular at least 0.4%, and most preferably at least about 0.8% on weight fabric of the textile material.

In preferred embodiments, chitosan is adhered to the textile material in an amount of at most 1.5%, preferably at most 1.0%, more preferably at most 0.7%, in particular at most 0.5%, and most preferably at most about 0.3% on weight fabric of the textile material. Furthermore, chitosan is adhered to the textile material in an amount of preferably at least 0.05%, more preferably at least 0.1%, even more preferably at least 0.2%, and most preferably at least 0.3% on weight fabric of the textile material.

In preferred embodiments, quaternary ammonium organosilane compounds are adhered to the textile material in an amount of at most 0.8%, preferably at most 0.4%, more preferably at most 0.3%, in particular at most 0.2%, and most preferably at most about 0.07% on weight fabric of the textile material. Furthermore, the quaternary ammonium organosilane compounds are adhered to the textile material in an amount of preferably at least 0.01%, preferably at least 0.02%, more preferably at least 0.04%, and most preferably at least about 0.07% on weight fabric of the textile material. The inventor found that if quaternary ammonium organosilane is applied to synthetic textile materials such as polyester or polyamide, amounts of more than 0.15% decrease the possibility to adhere hydrophilic and/or stain release agents in a subsequent processing step in a wash-durable manner. For cellulosic textiles such as cotton or viscose, the ability to bond hydrophilic and/or stain release agents in a wash-durable manner decreases even at lower amounts. Therefore, the use of quaternary ammonium organosilane is not preferred for cellulosic textiles where hydrophilic and/or stain release agents are to be adhered to the textile as well.

In particularly preferred embodiments, further described in the examples section below, in particular where the textile material comprises a cellulosic textile material,
- azole-based compounds are adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- metal is adhered to the textile material in an amount of at least 0.0002%, preferably at least 0.0005%, more preferably at least 0.001%, particularly at least 0.0013%, most preferably at least about 0.0017%, and/or in an amount of at most 0.02%, preferably at most 0.01%, more preferably at most 0.005%, particularly at most 0.003%, most preferably at most about 0.0017% on weight fabric of the textile material, and/or
- PHMB is adhered to the textile material in an amount of at least 0.05%, preferably at least 0.1%, more preferably at least 0.2%, particularly at least 0.4%, and most preferably at least about 0.6%, and/or in an amount of at most 2.0%, preferably at most 1.5%, more preferably at most 1.0%, particularly at most 0.8%, most preferably at most about 0.6% on weight fabric of the textile material.

In other particularly preferred embodiments, in particular where the textile material comprises a blend of cellulosic textile material and synthetic textile material,
- azole-based compounds are adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- metal is adhered to the textile material in an amount of at least 0.001%, preferably at least 0.002%, more preferably at least 0.004%, particularly at least 0.006%, most preferably at least about 0.0085%, and/or in an amount of at most 0.1%, preferably at most 0.05%, more preferably at most 0.03%, particularly at most 0.017%, most preferably at most about 0.0085% on weight fabric of the textile material, and/or
- PHMB is adhered to the textile material in an amount of at least 0.05%, preferably at least 0.1%, more preferably at least 0.2%, particularly at least 0.3%, and most preferably at least about 0.4%, and/or in an amount of at most 1.5%, preferably at most 1.0%, more preferably at most 0.8%, particularly at most 0.6%, most preferably at most about 0.4% on weight fabric of the textile material.

In further particularly preferred embodiments, further described in the examples section below, in particular where the textile material comprises substantially no cellulosic textile material,
- azole-based compounds are adhered to the textile material in an amount of at least 0.1%, preferably at least 0.2%, more preferably at least 0.4%, particularly at least 0.6%, and most preferably at least about 0.8%; and/or in an amount of at most 2.5%, preferably at most 2.0%, more preferably at most 1.5%, particularly at most 1.2%, most preferably at most about 0.8% on weight fabric of the textile material, and/or
- metal is adhered to the textile material in an amount of at least 0.001%, preferably at least 0.002%, more preferably at least 0.004%, particularly at least 0.006%, most preferably at least about 0.0085%, and/or in an amount of at most 0.1%, preferably at most 0.05%, more preferably at most 0.03%, particularly at most 0.017%, most preferably at most about 0.0085% on weight fabric of the textile material, and/or
- quaternary ammonium organosilane compounds are adhered to the textile material in an amount of at least 0.005%, preferably at least 0.01%, more preferably at least 0.02%, particularly at least 0.04%, and most preferably at least about 0.07%, and/or in an amount of at most 0.5%, preferably at most 0.3%, more preferably at most 0.2%, particularly at most 0.15%, most preferably at most about 0.07% on weight fabric of the textile material.

### Antimicrobial efficiency

The one or more antimicrobial agents adhered to the textile material preferably increase the reduction value of the textile material compared to the starting textile material, and/or the treated textile material preferably exhibits a reduction value of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 measured in accordance with AATCC test method 100-2012 and/or ASTM E2149-10 by/of at least 90% (1 log), preferably at least 99% (2 log), more preferably at least 99.9% (3 log) within 10 minutes of contact time and/or of at least 99% (2 log), preferably at least 99.9% (3 log), more preferably at least 99.99% (4 log) within 1 hour of contact time and/or of at least 99% (2 log), preferably at least 99.9% (3 log), more preferably at least 99.99% (4 log), most preferably at least 99.999% (5 log) within 24 hours of contact time.

Furthermore, a textile material according to preferred embodiments of the invention exhibits after 25 laundry washes a reduction value of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 of at least 99%, preferably at least 99.9%, within 10 minutes on continuous reinoculations followed by dry and wet alternate abrasion cycles, preferably when tested in accordance with EPA protocol 90072PA4 as modified, a custom protocol which is defined below. Preferably, the textile material passes the EPA protocol 90072PA4 as modified.

As described in more detail below, textiles according to preferred embodiments of the invention can be used as surface sanitizer. When e.g. sanitizing a ceramic surface by wiping a textile material over the surface, wherein no liquid antimicrobial agent is used or leaches from the textile material applied to the surface and/or wherein at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material is released from the textile material onto the surface, in preferred embodiments a reduction of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 on the surface by at least 99% (2 log), preferably at least 99.9% (3 log), more preferably at least 99.99% (4 log), even more preferably at least 99.999% (5 log), particularly at least 99.9999% (6 log), preferably evaluated after 0, 15, and/or 60 minutes post wiping can be achieved. Preferably, the reduction can even be achieved when the textile material is dry during wiping it over the surface.

The reduction can preferably be achieved by wiping the textile material over the surface for at most 90 seconds, more preferably for at most 60 seconds, even more preferably for at most 30 seconds. Preferably, the reduction can be achieved by exercising an average pressure on at least parts of the textile by a hand of the user or other wiping tool of at most 250 g/cm², preferably at most 200 g/cm², more preferably at most 150 g/cm², most preferably at most about 120 g/cm² during wiping the textile material over the surface, and/or a maximum pressure of at most 500 g/cm², preferably at most 400 g/cm², more preferably at most 300 g/cm², most preferably at most about 200 g/cm².

### Hydrophilic Agents/Stain Release Agents

A great variety of hydrophilic agents/stain release agents can be fixed to a textile by using the process of the invention described below. Like the preferred antimicrobial agents, the hydrophilic agents/stain release agents are preferably non-ionic or cationic, but not anionic.

According to preferred embodiments of the invention, the one or more hydrophilic agents adhered to the textile material comprise at least one selected from the group consisting of carboxylic acid esters, polyester ether copolymers, starch based emulsions, fatty alcohol ethoxylates, and organosilane terpolymers, preferably fatty alcohol ethoxylates or organosilane terpolymers, most preferably organosilane terpolymers.

According to preferred embodiments of the invention, the one or more stain release agents adhered to the textile material comprise at least one selected from the group consisting of fatty alcohol ethoxylates and organosilane terpolymers, preferably organosilane terpolymers. In this case, the one or more or all of the stain release agents can convey hydrophilic properties to the textile as well, which for many applications like surface sanitizers or cleaning wipes is particularly advantageous.

Organosilane terpolymer is known in the art and commercially available, e.g. in Hydrosil 8900, of Britacel Silicones Ltd., India. It is based on modified silicone and applicable for both natural (cellulosic) as well as synthetic textiles.

Fatty alcohol ethoxylates are known in the art and commercially available, e.g. in Hydroperm of Archroma, which contains ultra-fine polyester granules and is most suitable for cellulosic textiles, or in Permalose of Croda Chemicals, India, which contains ultra-fine polyurethane granules and is most suitable for synthetic textiles like polyester.

Both organosilane terpolymer and fatty alcohol ethoxylates are in preferred embodiments covalently bonded, or strongly fixed, optionally by using an inbuilt binder, to the textile material such that they can withstand numerous washes.

In preferred embodiments, the hydrophilic/stain release agents, in particular organosilane terpolymer and/or fatty alcohol ethoxylates are bonded without the use of a crosslinker, in particular without the use of isocyanate, more particularly blocked isocyanate

It is one merit of the invention to have recognized that the usability of many textiles such as surface sanitizers, cleaning wipes or other hygiene products can be increased by improving the hydrophilic properties of the textiles, which makes it easier to use the textiles together with water or other aqueous solvents. It is another merit of the invention to have recognized that the washability of such textiles can be increased by improving the stain release properties of the textiles. It is another merit of the invention to have identified several highly effective hydrophilic and stain release agents that achieve these properties. It is a further merit of the invention to have identified a process by which hydrophilic/stain release properties and in particular both antimicrobial and hydrophilic/stain release properties can be conveyed to a textile material, be it in one or more liquor application cycles, in a wash-durable or even substantially non-leaching manner. Finally, it is a merit of the invention to have identified combinations and amounts of antimicrobial agents and hydrophilic/stain release agents to be adhered to a textile material such that the hydrophilic/stain release agents do not prevent a satisfactory antimicrobial efficiency of the textile material, and the antimicrobial agents do not prevent satisfactory hydrophilic/stain release properties of the textile material.

### Amounts of hydrophilic agents/stain release agents adhered to the textile material

In preferred embodiments, the hydrophilic and/or stain release agents are adhered to the textile material in an amount of together at most 5%, preferably at most 4%, more preferably at most 3%, even more preferably at most 2%, in particular at most 1.5%, and most preferably at most about 1% on weight fabric of the textile material, and/or in an amount of together at least 0.1%, preferably at least 0.2%, more preferably at least 0.4%, and most preferably at least about 0.6% on weight fabric of the textile material.

Organosilane terpolymers are preferably adhered to the textile material in an amount of at least 0.1%, preferably at least 0.2%, more preferably at least 0.5%, particularly at least 0.7%, and most preferably at least about 1% on weight fabric of the textile material. Fatty alcohol ethoxylates are preferably adhered to the textile material in an amount of at most 3%, preferably at most 2%, more preferably at most 1.5%, even more preferably at most 1%, and most preferably at most about 0.7% on weight fabric of the textile material. The inventor found that when more than 1% o.w.f. of organosilane terpolymers or more than 0.7% (cellulosic textiles) or 0.4% (synthetic textiles) o.w.f. of fatty alcohol ethoxylates are adhered to a textile material, the efficiency of any antimicrobial agents adhered to the textile starts to decrease.

### Efficiency of the hydrophilic agents/stain release agents

In preferred embodiments of the invention, the one or more hydrophilic agents adhered to the textile material reduce the water absorbency time of the starting textile material by at least 20%, preferably at least 40%, more preferably at least 60%, and most preferably at least 80%, preferably when measured in accordance with AATCC test method 79-2014 (Option A). The treated textile material preferably exhibits a water absorbency time of at most 5 seconds, preferably at most 3 seconds, more preferably at most 2 seconds, and most preferably at most 1 second, preferably when measured in accordance with AATCC test method 79-2014 (Option A).

In some embodiments, the one or more hydrophilic agents adhered to the textile material preferably do not increase the water repellency of the starting textile material, measured in accordance with AATCC test method 22-2014. The treated textile material preferably exhibits a water repellency rating of at most 50, preferably 0, measured in accordance with AATCC test method 22-2014.

In some embodiments, the one or more hydrophilic agents adhered to the textile material increase the vertical wicking rate of the starting textile material by at least a 10%, preferably at least 20%, and most preferably at least 30%, when tested according to AATCC test method 197-2013. The treated textile material preferably exhibits a vertical wicking rate of at least 0.15 mm/sec, preferably at least 0.18 mm/sec, more preferably at least 0.20 mm/sec, and most preferably at least 0.23 mm/sec, when tested according to AATCC test method 197-2013. The vertical wicking rate is the speed at which liquid travels along or through a textile held vertically.

In some embodiments, the one or more hydrophilic agents adhered to the textile material increase the horizontal wicking rate of the starting textile material by at least 50%, preferably 100%, more preferably 300% and most preferably 500% when tested according to AATCC test method 198-2013. The treated textile material preferably exhibits a horizontal wicking rate of at least 15 mm²/sec, preferably at least 20 mm²/sec, more preferably at least 25 mm²/sec, and most preferably at least 30 mm²/sec, when tested according to AATCC test method 197-2013. The horizontal wicking rate is the change in the area of the liquid with respect to time as the liquid travels through a textile held horizontally.

In some embodiments, the one or more hydrophilic agents adhered to the textile material have the effect that the textile material becomes noticeably more easy to squeeze out than the starting textile material, which is particularly advantageous if the textile is used together with water or other solvents.

In some embodiments, the one or more stain release agents adhered to the textile material increase the stain release rating of the starting textile material by at least one grade, more preferably by at least two grades, particularly at least three grades, and most preferably 4 grades, when tested according to AATCC test method 130-2010. The treated textile material preferably has a stain release rating, measured in accordance with AATCC test method 130-2010, of at least grade 3, preferably at least grade 4, and most preferably grade 5.

In some embodiments, the one or more stain release agents adhered to the textile material increase the stain/oil repellency rating of the starting textile material, measured in accordance with AATCC test method 118-2013, by at most one grade, preferably do not increase the stain/oil repellency. The treated textile material preferably exhibits a stain/oil repellency rating, measured in accordance with AATCC test method 118-2013, of at most grade 1, preferably grade o.

### Starting Textile Material

Generally, any textile material can be used as the starting textile material if it comprises functional groups having the ability to bond one or more antimicrobial agents to the textile material. According to some embodiments of the invention, the starting textile material comprises hydroxyl, peptide and/or carbonyl groups, in particular hydroxyl and/or peptide. These functional groups enable fixing, bonding, attaching or adhering of one or more antimicrobial and/or hydrophilic or stain release agents to the textile material. In exemplary embodiments, the starting textile material comprises peptide and/or hydroxyl groups, in particular hydroxyl groups. According to the preferred embodiments of the invention, the textile material is a cellulosic textile material, a synthetic textile material, or a blend comprising a cellulosic textile material and a synthetic textile material. According to specific embodiments of the invention, the cellulosic textile material comprises cotton, viscose, rayon, linen, hemp, ramie, jute, and combinations (blends) thereof, preferably cotton or viscose or combinations thereof.

Examples of embodiments of synthetic textile material include polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester. Blends of cellulosic and synthetic textile materials comprise between 20% and 80% of cellulosic textile material, preferably between 30% and 75%, more preferably between 50% and 70%, and most preferably about 60%. In specific embodiments the blend of cellulosic textile material and synthetic textile material comprises between 20 and 80% of synthetic textile material, preferably between 25 and 70%, more preferably between 30 and 50%, and most preferably about 40%.

Synthetic textiles are typically stronger and more durable than most textiles made of natural fibers. In addition, it is typically easier to bind chemical substances to a synthetic textile. Counterexamples are the antimicrobial agents PHMB and chitosan, which are difficult to adhere to synthetic textiles. Synthetic textiles can be designed to not wrinkle, and they typically offer a priori stretching and stain release functionality. However, they are typically hydrophobic in nature and hardly biodegradable. Depending on the requirements, these functionalities may also be disadvantageous. Natural textiles are, in turn, fairly biodegradable and hydrophilic in nature. The advantages of synthetic and natural textiles tend to be opposing. Wrinkling, stain release and hydrophobic/hydrophilic properties may be mentioned as examples.

The aforementioned textile materials may be textile material selected from the group consisting of woven, knitted, warp-knitted, crocheted, and non-woven fabrics.

A preferred starting textile material of the invention has a water holding capacity of at least 4 times its weight, preferably at least 6 times its weight, more preferably at least 7 times its weight, in particular at least 8 times its weight, most preferably at least 9 times its weight, preferably when tested according to ASTM D7367-14.

The skilled person understands that several of the components listed above may be combined and thereby form the starting textile material to be finished with process 10 described subsequently.

In general, any starting textile material can be processed with said process 100, wherein the textile material is a fiber, preferably a yarn or a fabric, and most preferably a fabric. In case the textile material is a fabric, it can generally have any specific weight, or fabric weight, such as e.g. 100, 200 or 300 g/m² (GSM)

### Finishing Process

### Process Cycles

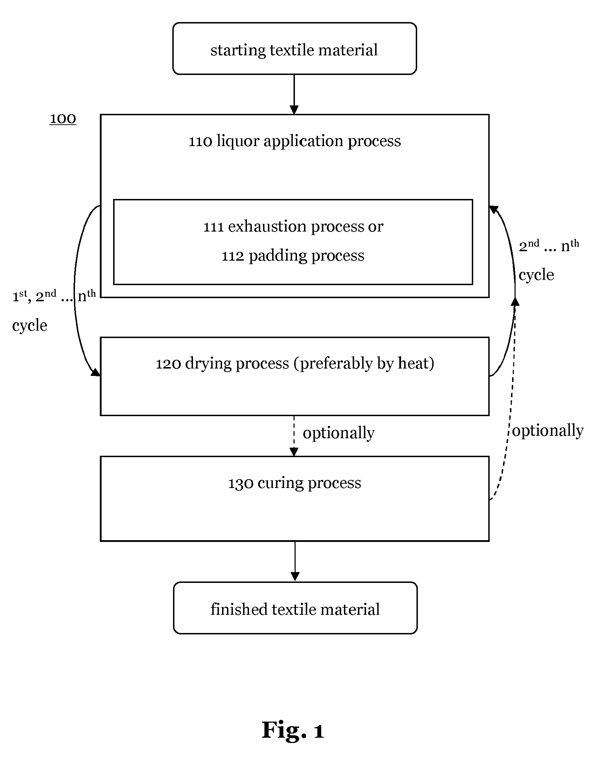

The preferred process of finishing a textile material according to the present invention comprises one or more process cycles 100 of adhering antimicrobial and/or hydrophilic/stain release agents to a textile material. Each process cycle 100, exemplarily shown in Fig. 1, can be divided into at least two process steps, a first process step 110 and a second process step 120. The first process step is a liquor application process. The liquor application process is followed by the second process step 120, in which the textile material is dried, preferably by subjecting it to a heat treatment. Optionally, the step of drying is followed by a third process step 130, in which the textile material is cured. At least the last cycle of the process of finishing a textile material according to the present invention should comprise the third step 130 of curing.

### Liquor Application Process

A liquor is a liquid containing chemicals to be applied to a textile. In the present invention, the liquor comprises one or more antimicrobial agents and/or one or more hydrophilic/stain release agents. A liquor application process is any process by which the textile is brought in contact with the liquor to treat the textile with the chemicals.

The liquor application process 110 shown in Fig. 1 comprises preferably an exhaust process 111 or a padding process 112.

Preferably, the liquor has a pH-value of at most 6.9, preferably at most 6.5, more preferably at most 6.3, in particular at most 6.0, and most preferably at most about 5.5, and/or a pH-value of at least 3.0, preferably at least 3.5, more preferably at least 4.0, even more preferably at least 4.5, in particular at least 5.0, and most preferably at least about 5.5.

The liquor contains a solvent, preferably water, and the agents and the solvent preferably form a homogenous mixture, and in particular do not form a slurry or dispersion. The antimicrobial and/or the hydrophilic/stain release agents and preferably all other components in the liquor are preferably dissolved in the liquor, in particular are they not dispersed in the liquor, and/or the liquor is substantially free of solids.

The dynamic viscosity of the liquor at 20 °C and/or 80 °C, in centipoise (cP), is preferably at most 20% higher than the dynamic viscosity of water at 20 °C and/or 80 °C, respectively, preferably at most 10%, more preferably at most 5%, particularly at most 2%, and most preferably at most about 0% higher. The amount of latex in the liquor is preferably at most 10 gpl, preferably at most 5 gpl, more preferably at most 2 gpl, even more preferably at most 1 gpl, and most preferably o.

The amount of cyclodextrin and/or inclusion complexes, in particular inclusion complexes of fiber-reactive cyclodextrin derivatives and antimicrobial agents is preferably at most 10 gpl, more preferably at most 5 gpl, even more preferably at most 2 gpl, particularly at most 1 gpl, and most preferably o. The amount of dye in the liquor is preferably at most 10 gpl, more preferably at most 5 gpl, even more preferably at most 2 gpl, particularly at most 1 gpl, and most preferably o.

### Exhaustion

As is known in the art, during an exhaust process (see e.g. step 111 in Fig. 1), a textile material is brought in contact with a liquor which comprises ingredients which are transferred to the textile material during the exhaust process. This can be achieved by guiding the textile material through a container filled with the liquor. Yarn and woven fabrics are typically treated with exhaust processes. During a common exhaust process, chemicals to be applied to a textile material are dissolved or dispersed in a solvent, e.g. water, according to the required material to liquor ratio, which describes the ratio between the weight of the textile to be treated and the weight of the liquor. For example, if the desired material to liquor ratio is 1:2, there would be 600 kg of liquor for 300 kg of textile material to be exhausted. The textile material is brought in contact with the liquor, for example by immersing it into the liquor, whereby the chemicals preferably contact the fibers and more preferably enter the fibers. For obtaining proper diffusion and penetration of the chemicals in the fiber, a respective liquor temperature and respective exhaustion time are set, such that kinetic and thermodynamic reactions take place as desired. As the textile material and its fibers absorb the chemicals, the concentration thereof in the liquor decreases. As is known in the art, the degree of liquor exhaustion as a function of elapsed time is termed extent of the exhaust process. The percentage of the chemicals initially present in the liquor which is exhausted onto the textile at the end of the process is called exhaustion rate or exhaust rate. Preferably, the exhaustion rate of the exhaust process is at least 90%, preferably at least 95%, more preferably at least 98%. This exhaustion rate allows for reducing costs, as most of the ingredients of the liquor are exhausted by the textile material. It is also more ecological than processes with lower exhaustion rates.

The exhaust process 111 shown in Fig. 1 may be performed by any suitable technique, and on any suitable machine, like a yarn dying machine, a beam machine, a winch machine, a jet-dyeing machine, a continuous dyeing range (CDR), continuous bleaching range (CBR), or a jigger machine. In a jigger machine, an open-width fabric revolves on two main rollers. The fabric passes from one roller through the liquor bath at the bottom of the machine and then onto a driven take-up roller on the other side. When all the fabric has passed through the bath, the direction is reversed. Each passage is called an end. The process typically involves an even number of ends. The liquor bath has one or more guide rollers around which the cloth travels. During the immersion, the desired contact with the process liquor is achieved. When passing through the liquor bath, the fabric picks up an adequate quantity of liquor, excess of which is drained out, but still a good quantity is held in the fabric. During rotation of the rollers, the chemicals contained in the liquor penetrate and diffuse into the fabric. The largest part of the diffusion process takes place not in the liquor bath but when the fabric is on the rollers, since only a very small length of fabric is in the liquor bath at a given time, and the major part is on the rollers. Jigger machines are preferred because they are very economical and because they can be used with a high material to liquor ratio.

The exhaust process 111 shown in Fig. 1 allows for evenly spreading the liquor across the entire cross section of the textile material, such that preferably no spot of the textile material is left untouched by the liquor. As a result, interactions and/or bonds may be created between the textile material and one or more agents at this time. Preferably, most of the one or more agents of the liquor are exhausted evenly onto the entire cross section of the textile material.

In general, more heat on the fabric is better for bonding. Therefore, preferably, the temperature of the liquor during the exhaust process is sufficiently high and the exhaust time is sufficiently long such that the one or more agents in the liquor are substantially uniformly dispersed across the cross section of the textile material as a result of the exhaust process. Thus, the temperature of the liquor should be sufficiently high and the exhaust time should be sufficiently long such that preferably the textile material is well impregnated and the one or more agents are dispersed throughout the entire textile material. Preferably, the exhaust time is sufficiently long and the temperature of the liquor during the exhaust process is sufficiently high such that the textile material can achieve the desired performance after a respective curing process, as will be outlined below.

However, too much heat causes yellowness and weakens the fabric. Therefore, preferably, the temperature of the liquor during the exhaust process is sufficiently low and/or the exhaust time is sufficiently short such that the textile material does not discolor and/or turn yellow and/or its breaking (tensile) strength is not reduced by more than 15%, preferably not more than 10%, more preferably not more than 7%, and most preferably not more than 5%, as a result of the exhaust process. At too high temperatures, too much steam forms, reducing the efficiency of the process. Furthermore, if the temperature of the liquor is too high, turbulences can occur within the liquor bath and the textile material may get harmed.

The term exhaust time when used in the context of the present invention is preferably defined as the period starting when at least part of the entire batch of textile material first comes into contact with the liquor and lasting until the last part of the batch is taken out of the liquor. For a given application, the ideal exhaust time can vary significantly. In case the textile is a fabric, it will depend on the type of machine, the size of the liquor bath, and the length and weight of the fabric. For example, if the ideal exhaust time for a fabric of a length of 1,500 meters is 60 minutes, the ideal exhaust time for a fabric of a length of 3,000 meters may be 100 minutes under otherwise identical conditions. Whenever an exhaust time is specified herein, it refers to the time which is equivalent to the exhaust time of a fabric of 1,500 meters in length and 200 g/m² in weight on a standard jigger machine (e.g. model number Y1100 manufactured by Yamuda) being operated at a standard fabric speed (e.g. 50 meters/minute). For any given textile material and exhaustion machine, the skilled person, using common general knowledge, will be able to determine the exhaust time which is equivalent to an exhaust time specified for the above-mentioned parameters.

The breaking strength may be measured with any suitable technique, and is preferably measured in accordance with ASTM standard D 5035-11 (in case the textile material is a fabric), or in accordance with ASTM standard D 2256/D 2256M-10e1 (in case the textile material is a yarn).

The inventors found that the preferred temperature of the liquor during the exhaust process and the exhaust time is substantially independent of the weight and the type of the textile material, and of the agents in the liquor. This is because the ideal exhaust process parameters are determined by the way textiles, in particular multifilament yarns and fabrics, behave in general.

In the preferred embodiments, the liquor has a temperature of at least 45 °C, preferably of at least 50 °C, more preferably of at least 60 °C, particularly of at least 70 °C, and most preferably of at least about 80 °C. Furthermore, the liquor has a temperature below boiling temperature, preferably of at most 95 °C, more preferably of at most 90 °C, particularly of at most 85 °C, and most preferably of at most about 80 °C. The exhaustion process time is preferably at most 90 min, preferably at most 80 min, more preferably at most 70 min, and most preferably at most about 60 min. Furthermore, the exhaustion process time is preferably at least 45 min, preferably at least 50 min, more preferably at least 55 min, and most preferably at least about 60 min. As shown in International patent application number PCT/EP2016/054245 by the same applicant, when a textile is treated at a temperature of 80 °C for 60 minutes, it expands and opens up, exposing individual fibers so that the agent can reach even the most remote spot, and there is even dispersion of the agents. Accordingly, different textile materials can easily be treated by means of the exhaust process 111 shown in Fig. 1 without having to change parameters of the exhaust process, while still obtaining the best possible results.

Preferably, during the exhaust process 111 shown in Fig. 1, the liquor is stirred. The stirring should be performed at intervals, in other words, the stirring is performed regularly during the exhaust process with interruptions. It will be appreciated that other suitable intervals may preferably be set, depending on the specific application. Ideally, the stirring is performed continuously during the exhaust process. This intermixing of the chemicals in the exhaust bath increases reliability of the exhaust process, as one or more agents are more evenly distributed in the bath and as a result, a product with even quality throughout the entire textile material can be obtained. Preferably, the stirring is performed by means of a circulation pump, which circulates the liquor inside the exhaustion bath and which is typically comprised by a conventional exhaustion machine. The stirrer used by the inventors is a simple mixer, which is similar to but larger than a standard household mixer. The stirrer was added by the inventors to the exhaustion machine they used as it is not provided by conventional exhaustion machines. Most preferably, the liquor is stirred by means of both a circulation pump and a stirrer. Due to this extensive mixing of the liquor, the exhaust process is supported and one or more agents are well dispersed across the cross section of the textile material during the exhaust process. As is known in the art, an exhaust process is typically applied for dyeing cloth, for example. In such applications, typically only a circulation pump is applied for ensuring proper fluid characteristics of the bath, such that a homogeneous dispersion of the dyeing molecules is present in the bath. However, since the one or more agents used in the context of the present invention can be less soluble in water compared to dyeing agents, the utilization of both a stirrer and a circulation pump assures that the agents are not undissolved and do not settle at the bottom of the bath. Instead, due to the combination of both stirring means, the agents are uniformly and homogeneous dispersed throughout the bath.

Accordingly, with exhaust process 111 shown in Fig. 1, agents are substantially uniformly dispersed across the cross section of the textile material, whereby the textile material itself, advantageously, does not yellow and essentially does not lose its breaking strength.

### Padding

Any suitable technique can be utilized for performing a padding process 112 shown in Fig. 1, in which preferably a respective liquor is prepared and fed through a pump to a respective padding mangle. Accordingly, padding process 112 shown in Fig. 1 preferably comprises applications of one or more rolls to obtain optimum wet pickup of the liquor on the textile material. According to a preferred embodiment, the textile material passes through a multiple trough padding mangle, or a continuous dyeing or bleaching range. The liquor may be at room temperature or it may be heated during the padding process.

The appropriate padding mangle pressure is typically predetermined, depending on the quality of the textile material, and it is in general set such that the pick-up rate (or "wet pick-up") of the agents is optimized. The pick-up rate specifies the amount of liquor applied and is defined as a percentage on the weight of the dry untreated textile as follows: % pick-up rate = weight of liquor applied x 100 / weight of dry textile. For example, a pick-up rate of 65% means that 650 grams of liquor are applied to 1 kg of textile. Preferably, a liquor pickup rate of the padding process is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%. However, if the textile is already to a certain extent saturated with agents before the padding process is applied, e.g. because another process cycle has been performed previously, it is believed that the effective pick-up rate for the agents is only about 70% of the nominal pick-up rate mentioned above, in the sense that the rest of the agents padded onto the fabric does not become permanently fixed to the fabric.

### Drying

Any suitable technique can be utilized for performing the drying process step 120 shown in Fig. 1. Drying is, however, preferably performed by subjecting the textile material to a heat treatment. The heat treatment is preferred because it is more efficient for various reasons: accelerated manufacturing, i.e. shortened residence time of the textile material in the production chain and lean process management without removal for drying purposes and re-insertion of the textile material in the processing chain may be mentioned as examples. Furthermore, the textile should be dried by a heat treatment before it is washed. This is because the heat treatment will achieve basic bonding of the agents to the textile so that they will not immediately be washed out during the washing process.

After drying process 120 by heat treatment, the textile material should be 99% devoid of moisture. However, when the textile cools down to room temperature, it will have a moisture regain of, e.g., about 7-8% for cotton and of about 4-5% for polyester.

Preferably the drying is conducted at least partially at an ambient temperature of at least 70 °C, preferably at least 100 °C, more preferably at least 110 °C, most preferably at least about 120 °C. Lower temperatures would require longer dwell time, which is disadvantageous because a longer dwell time has a negative impact on the textile in terms of yellowing and also on the strength of the fabric. Furthermore, the drying is preferably conducted at an ambient temperature of at most 160 °C, preferably of at most 140 °C, more preferably of at most 130 °C, and most preferably of at most about 120 °C.

Preferably, the drying time at the temperatures given above is of at least 30 seconds, preferably at least 40 seconds, more preferably at least 50 seconds, and most preferably at least about 60 seconds, per 100 g of fabric weight per m² (in case the textile material is a fabric). Further preferably, the drying is performed over a period of at most 120 seconds, preferably at most 90 seconds, more preferably at most 75 seconds, most preferably at most about 60 seconds, per 100 g of fabric weight per m² (in case the textile material is a fabric). It will be appreciated that the drying times increase with increasing fabric weight (per m²). The skilled person understands that similar drying times apply if the textile material is a yarn, and understands to choose respective drying times which then depend on the yarn diameter.

Drying process 120 is typically conducted by passing the textile material through a stenter or stenter frame (sometimes also referred to as a "tenter") or similar drying machine. By drying the textile material, preferably excess moisture is removed.

### Curing

A curing process in the context of the present invention (see e.g. 130 in Fig. 1) is essentially a heat treatment process applied to a textile material for inducing physical cross-linking. Curing can only be performed once the textile is dry because the temperature of the textile cannot exceed 100 °C until the water in the textile is evaporated.

The curing temperature is preferably sufficiently high and the curing time is preferably sufficiently long such that one or more agents applied to (preferably exhausted and/or padded onto) the textile material are sufficiently strongly fixed or bonded to the textile material. They should preferably be set such that the agents are bound to the textile material and optionally polymerized, become an inherent part of the textile material and provide the desired properties of the textile material in a wash-durable or even non-leaching manner. Depending on the agents and chemicals used, also a large part of the crosslinking of the agents takes place during the curing step. In case the textile material is a fabric, the curing time depends on the weight of the fabric (per m²). However, the inventors found that the preferred curing temperature, which will be detailed below, is substantially independent of the type of the textile material.

Furthermore, the curing temperature is sufficiently low and the curing time is sufficiently short such that the textile material does not discolor and/or turn yellow, and/or its breaking strength is not significantly reduced. Further preferred, the curing temperature is sufficiently low and the curing time is sufficiently short such that the textile material does not melt and/or burn and/or yellow, and/or that the colors of the textile material do not substantially change (discolor) as a result of the curing.

In the preferred embodiments, the curing process is conducted at least partially at an ambient curing temperature of at least 140 °C, preferably at least 160 °C, more preferably at least 170 °C, particularly at least 175 °C, and most preferably at least about 180 °C. Preferably, the curing process is conducted at an ambient temperature of at most 200 °C, more preferably at most 190 °C, even more preferably at most 185 °C, and most preferably at most about 180 °C. Thus, in the most preferred embodiment, the maximum curing temperature is 180 °C, independent from the textile material treated with process 100.

In particular where the textile material is a fabric of less than 350 grams per m², curing takes place at the above stated curing ambient temperatures over a period of preferably at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

Where the textile material is a fabric between 350 and 500 grams per m², curing takes place at the above stated curing ambient temperature over a period of preferably at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

Where the textile material is a fabric of at least 500 grams per m², curing takes place at the above stated curing ambient temperature over a period of preferably at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds.

The preferred process parameters for curing (at 180 °C during the curing times mentioned above) were obtained in an extensive research and development campaign performed by the inventor and described in International patent application PCT/EP2016/054245 by the same applicant.

The curing process 130 is preferably carried out in a pass through a stenter. Referring to Fig. 1, a drying process 120 following a liquor application process may be directly followed by a curing process 130. In this case, the drying process 120 and the curing process 130 is preferably carried out together with one single pass through the stenter, preferably with a temperature ramp-up and ramp-down procedure as described in International patent application PCT/EP2016/054245 by the same applicant. Furthermore, where the textile material is a fabric, drying and curing of the textile material are performed at the above stated drying/curing ambient temperatures over a period of together at least 45 seconds, preferably at least 50 seconds, more preferably at least 55 seconds, most preferably at least about 60 seconds, per 100 grams of fabric weight per square meter, and preferably at most 75 seconds, preferably at most 70 seconds, more preferably at most 65 seconds, most preferably at most about 60 seconds, per 100 grams of fabric weight per square meter.

### Putting the cycles together

The subsequent sections are dedicated to the description of the preferred and most preferred embodiments based on the above stated elementary building blocks specifying the starting textile materials, composition of liquors as well as processing parameters including concentrations of agents, temperatures and processing times at standard pressure.

Exhaustion is preferred over padding in the first process cycle if the starting textile material is a multifilament yarn or a fabric made out of them, which is preferred for most applications because multifilament textiles are stronger, have a higher surface area, and can be blended. In an exhaust process, the multifilament textile opens up and the fibers are individually exposed to penetration by the agents. Thus, by use of an exhaust process, the agents can diffuse into the fibers and do not occupy the surface space of the fibers to the same extent as it is the case in more superficial liquor application processes like padding or spraying.

The use of an exhaust process in the first process cycle is particularly advantageous in cases where the first process cycle is followed by a further process cycle. The use of an exhaustion process in the first process cycle allows to improve the performance or convey another property by a second process cycle, in particular by a second process cycle in which a padding process is used. E.g., the inventor found in experiments, whose results are not reproduced herein, that the antimicrobial performance and/or the wash-durability thereof can be increased if the textile material is treated with an antimicrobial liquor application process in each of two process cycles. In this case, the first antimicrobial liquor application process is preferably an exhaust process, and the second liquor application process is preferably a padding process. The two antimicrobial cycles can then be followed by one or more cycles in which a hydrophilic/stain release liquor application process is performed.

In contrast, repeated superficial liquor applications like repeated padding applications will not improve performance, or at least not to the same extent, and it will be more difficult to impart another property in the second cycle. Furthermore, the inventors found that leaching is at lowest values only when exhaustion is used in the first process cycle. On the other hand, in the case of non-woven fabrics, padding is preferred because non-woven fabrics can oftentimes not withstand the forces applied by exhaustion machines like jiggers.

For embodiments which make use of more than one processing cycle, padding is preferred for all subsequent cycles, independent of the starting textile material. This is because once a textile has been exhausted with agents in a first process cycle, the interior of the fibers is saturated at least to a certain degree, and space for more agents to be adhered to the textile material will be found primarily on the fiber surface. Furthermore, padding is less time consuming and therefore less costly than exhaustion.

Curing, which consumes large amounts of energy and decreases the breaking strength of the textile considerably, is preferably only performed in the last cycle. This is in general sufficient for bonding even the agents applied in the previous cycles to the textile. Some embodiments may comprise a washing step at the end of one or more or even all cycles, to obtain particularly low leaching values (for a detailed description see International patent application PCT/EP2016/054245 by the same applicant). However, this may not be necessary for most of the preferred applications disclosed herein, such as wipes and other sanitary products. Where washing is carried out after the drying step, in particular without or before curing, the drying should be performed with a heat treatment, preferably at least at the above-specified temperatures for drying. The heat treatment will ensure that there is a basic bonding between the textile and the agents applied in this cycle, which prevents washing out of these agents in the washing step.

As a rule, where both antimicrobial and hydrophilic/stain release agents are applied to the starting textile material, antimicrobial and hydrophilic/stain release agents are, on the one hand, separately applied, i.e. in separate cycles. On the other hand, the antimicrobial agents are applied in one or more cycles prior to the cycle in which hydrophilic/stain release agents are applied, or applied for the first time. The inventor found that it is difficult, if not impossible to apply antimicrobial agents and hydrophilic/stain release agents in the same liquor in a wash-durable manner, or to apply antimicrobial agents in a wash-durable manner after hydrophilic/stain release agents have been applied to the textile. Metal, in particular silver is an exception to this rule, as it may be applied together with hydrophilic/stain release agents in the same liquor, i.e. in the same cycle. However, it should not be applied after the application of hydrophilic/stain release agents, as the pores of the textile would be blocked and bonding would be severely hampered.

A first group of embodiments of the present invention comprises the finishing of starting textile materials by adhering only antimicrobial agents to them but no hydrophilic/stain release agents, in one or more cycles of the finishing process of the present invention. A second group of embodiments comprises the finishing of starting textile materials by adhering only hydrophilic/stain release agents to them but no antimicrobial agents, in one or more cycles of the finishing process of the present invention. However, the most preferred group of embodiments of the present invention comprises the application of both antimicrobial agents and hydrophilic/stain release agents, in two separate cycles.

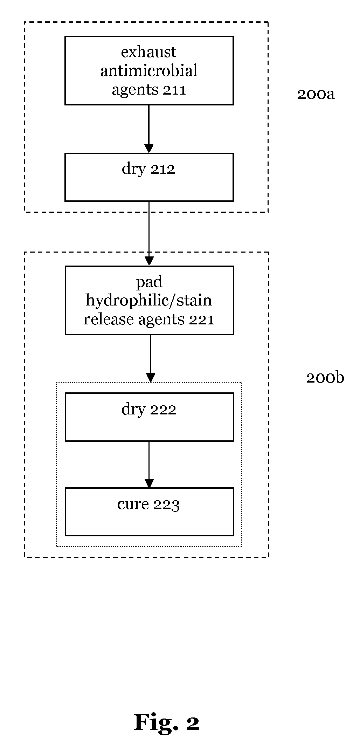

The preferred process of adhering both antimicrobial agents and hydrophilic/stain release agents to a textile material will now be described with reference to Fig. 2. In a first cycle 200a, the starting textile material, preferably a woven fabric, is subjected to an exhaustion process 211, wherein the liquor comprises one or more antimicrobial agents dissolved in water, with the pH-value adjusted as specified above. The selection and the concentration of the antimicrobial agents in the liquor are such that the exhaustion process 211 results in a textile material to which the agents as specified above are adhered in the combinations and the amounts as specified above. Exhaustion 211 is performed at standard pressure with an exhaustion temperature of 80 °C for 60 minutes while the liquor is stirred. The first process cycle 200a is finished by drying 212 as described above, at 120 °C.

In a second cycle 200b, the textile material is subjected to a padding process 221, wherein the liquor comprises hydrophilic/stain release agents dissolved in water, with the pH-value again adjusted as specified above. The selection and the concentration of the hydrophilic/stain agents in the liquor are such that the padding process 221 results in a textile material to which the agents are adhered in the combinations and the amounts as specified above. Padding 221 is performed at both standard pressure and temperature, with a liquor pickup rate of 100%. The second process cycle is finished by drying 222 followed by curing 223 at 180 °C, wherein drying and curing are performed in one single pass through a stenter, which provides the finished textile material.

It will be appreciated that one or more additional process steps or cycles may be introduced between the individual process steps or cycles of process 100 of Fig. 1. Furthermore, one or more additional process steps or cycles may be performed prior to or after performing process 100 of Fig. 1. For example, before the start of process 100 with the liquor application process 110, the textile material should be tested, washed and/or cleaned. Preferably, the fabric is first tested and if necessary washed or cleaned, so that the fabric is free from all chemical contaminants that would hinder the application of the chemistry to the textile. In a particularly preferred embodiment, one or more of the following steps may be performed prior to conducting process 100 of Fig. 1: Testing the textile material at laboratory scale to verify and confirm that it meets respective selection criteria, batching and stitching together of individual textile pieces on a frame, inspecting the textile material thoroughly for defects, ensuring that the fabric is free from any chemical contaminants.

The textile material may be dyed prior to performing process 100. In some embodiments, the textile material is manufactured to be multifunctional. After having performed process 100, i.e. after the antimicrobial and/or hydrophilic/stain release treatment, a respective multifunctional treatment is performed. With such a multifunctional treatment, the textile material may be provided e.g. with UV-blocking and/or other properties. It is also possible to conduct a multifunctional treatment in a padding process 112, wherein the padding liquor contains the respective functional agents, in addition to antimicrobial or hydrophilic/stain release agents.

It will be appreciated that different machines may be utilized in case the textile material is a yarn. For example, the exhaustion process may be performed with a pressurized yarn dyeing machine, and the yarn may then be treated with a hydro extractor for removing excess moisture. The drying and curing of the yarn may take place in a Radio Frequency RF Dryer and curing machine. The dwell times thereby depend on the yarn diameter, wherein the temperatures mentioned above still apply.

### EXAMPLES

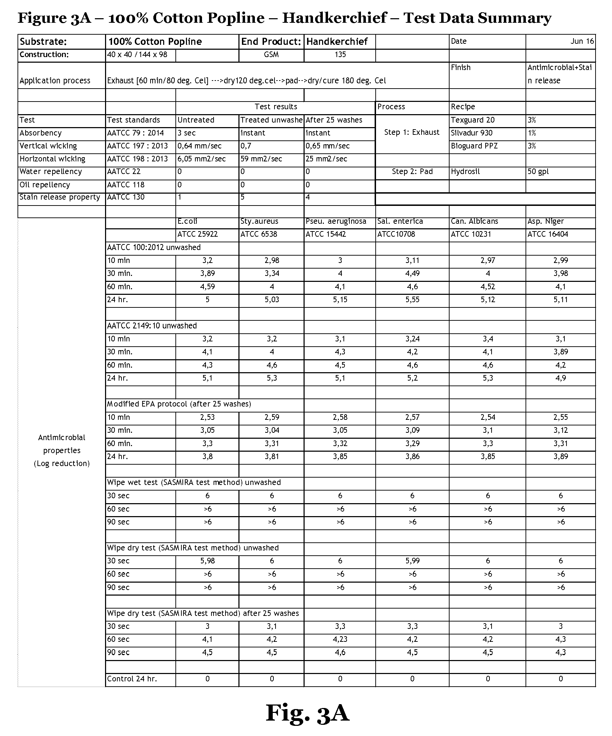

A series of different textile fabrics and materials were finished and tested according to the test protocols stated herein. The finishing process and the test results as well as the references to the underlying test protocols are summarized in the tables of Fig. 3A to Fig. 13C. All finished textile fabrics and materials were treated with antimicrobial agents and can be used as surface sanitizers.

The finishing processes are summarized on the upper side of each table under the section "application process". Fabrics were produced under laboratory conditions closely simulating the finishing processes described below.

### Chemicals

Chemicals used for the examples and mentioned in the tables comprise the following:
"Texguard 20" of Swissol Specialties Pvt. Ltd, India A: a solution containing 20% PHMB;
"Biogard PPZ" (=Biogard PPZ 250) of Beyond Surface Technologies, Switzerland: a solution containing 25% of propiconazole;
"Silvadur 930" of Dow Chemical Company, USA: a solution containing 0.17% of silver cations trapped in a matrix;
"AEM 5772" of Aegis, USA: a solution containing 72% of quaternary ammonium organosilane compounds;
"Hydrosil" (= Hydrosil 8900) of Britacel Silicones Ltd., India: a solution containing 30% organosilane terpolymer;
"Permalose" of Croda Chemicals, India: a solution containing 12% of fatty alcohol ethoxylates.

Recipes are stated in the figures. Percentages refer to the weight of the chemicals relative to the weight of the textile material ("o.w.f.") if not otherwise stated. The percentages of active agents which were used can be calculated with the concentration of actives in the chemicals as specified above. E.g., 3% Texguard 20 means that 20% x 3% = 0.6% o.w.f. of PHMB were used. The expression "gpl" refers to the amount of a chemical in a liquor. Again, the amount of actives can be calculated with the concentration of actives in the chemicals as specified above. E.g., 50 gpl Hydrosil means that 30% x 50 gpl = 15 gpl of organosilane terpolymer were comprised in a liquor. Other units are explicitly stated in each table.

### Test protocols

The underlying test protocols are generally publically available from the American Association of Textile Chemists and Colorist (AATCC) and the American Society for Testing and Materials (ASTM).

All microbiological tests were performed with cultures of the bacteria *Escherichia coli* American Type Culture Collection (ATCC) 25922, *Staphylococcus aureus* ATCC 6538, *Pseudomonas aeruginosa* ATCC 15442, *Salmonella enterica* ATCC 10708, and of the fungi *Candida albicans* ATCC 10231, and *Aspergillus Niger* ATCC 10231.

The SASMIRA wipe test is a custom wipe test in which a sterilized ceramic surface of about 10 cm × 10 cm is spiked with a test culture made in a nutrient broth of double strength with a certain inoculum strength (number of colony forming units per milliliter of the test culture). The culture is allowed to settle on the ceramic surface for one hour. Samples of the textile material are prepared including sterilization through exposure to UV radiation for 15 minutes. Dry samples of the sterile textile material are then wiped using the three middle fingers of one hand over the incubated ceramic surface for a total contact time of 30, 60 or 90 seconds at 60 Hz wiping frequency with an average wiping pressure of 120 g/cm² and a maximum wiping pressure of 200 g/cm². All areas of the ceramic surface were covered by the wiping in regular intervals. The remaining culture on the wiped ceramic surface is analyzed (1) immediately and (2) 60 minutes post wiping using ISO swab test method 18539:2004.

"EPA protocol 90072PA4" is a custom-specified test method based on AATCC test method 100-2012 and was set-up by Healthprotex, LLC, USA together with the United States Environmental Protection Agency (EPA). It was labelled with code 90072PA4 by the EPA. The specification of the protocol is reproduced below:
*** START OF SPECIFATION OF THE PROTOCOL ***

### Title: Healthprotex, LLC Protocol for Evaluating the Antimicrobial Efficacy of Textiles - Test Method for Evaluating the Antimicrobial Efficacy Textiles

Purpose: The purpose of this study is to document the efficacy of the test substance against the test system (microorganisms) under the test parameters specified in this protocol.

Method Reference: AATCC 100-2012 (Antibacterial Finishes: Assessment of). Note: This protocol describes a modified version of the aforementioned test method

### Test System (Microorganism):

Staphylococcus aureus ATCC 6538
Escherichia coli ATCC 11229
Pseudomonas aeruginosa ATCC 15442
Salmonella enterica ATCC 10708
Staphylococcus aureus (MRSA) ATCC 33592

### Study Parameters, Incorporated by Reference

**Non-Continuous Reduction Parameters:**

| | |
|---|---|
| Efficacy "Contact" Time: | ≤ 2 Hours |
| Unlaundered Control Textile Replicates: | 3 per test microorganism |
| Laundered Control Textile Replicates: | 3 per test microorganism |
| Unlaundered Treated Textile Replicates: | 3 per test microorganism/Lot |
| Laundered Treated Textile Replicates: | 3 per test microorganism/Lot |

**Non-Continuous Reduction Parameters (All Laundered):**

| | |
|---|---|
| Efficacy "Contact" Time: | ≤ 2 Hours |
| Abraded Control Textile Replicates: | 3 per test microorganism |
| Non-Abraded Control Textile Replicates: | 2 per test microorganism |
| Abraded Treated Textile Replicates: | 3 per test microorganism/Lot |

### Procedure:

Laundering, Environmental Stressing and Re-inoculation of Treated and Control Textiles
- A sufficient amount of each uncut treated and control textile is washed in a laundry washing machine at 85±15 °C using brand name non-antimicrobial, non-ionic and non-chlorine containing laundry detergent followed by a standard rinse cycle and dried at 62-96 °C for a period of 20-30 minutes.
- Laundered samples are placed in a 36 ± 2 °C incubator with a relative humidity of 85-100% for 2 hours (± 10 minutes) followed by exposure to UV by placing in a Class II biological safety hood for 15 ± 2 minutes at 20-25 °C with UV light on (treated and control fabric are laid flat to fully expose the fabric).

- After UV exposure, each carrier (treated and control) is inoculated with 0.100ml of re-inoculation culture to yield ≥1x104 CFU/Carrier, and allowed to sit undisturbed for 15±5 minutes at room temperature, at which time the next laundering cycle is initiated.
- See Preparation of Re-inoculation Culture Inoculum for details of re-inoculation culture preparation.
- The 25th cycle will not contain laundry detergent for the purpose of removing residual detergent from previous cycles and in preparation for efficacy testing, but will receive the heat, UV and re-inoculation mentioned above.

### Abrasion and Re-inoculation

- Treated and Control Carriers undergo a wear and re-inoculation regimen. A series of 12 abrasions and 11 reinoculations are performed and according to the table below. All abrasions and reinoculations are to be completed prior to the final efficacy evaluation test performed at least 24 hours after initial inoculation but not to exceed 48 hours. This step is performed at room temperature. The table below summarizes the manipulations of all carriers in the study.
- Abrasions are conducted between 45-55% relative humidity (RH). Temperature and room humidity measurements are taken and recorded periodically throughout the abrasion process.
- The weight of the fully assembled abrasion boats are recorded prior to initiation of the wear and re-inoculation regimen and must equal 1084 ± 1.0g.
- The abrasion tester is set to a speed of 2.25 to 2.5 for a total surface contact time of approximately 4-5 seconds, for one complete abrasion cycle.
- Each abrasion cycle in this test equals a total of 4 passes (e.g. left to right, right to left, left to right, and right to left).
- All surfaces in contact with carriers on the Gardner apparatus are decontaminated with absolute ethanol and allowed to dry completely between each set of surface wears to prevent carry over contamination.
- The foam liner and cotton cloths on the abrasion tester are replaced between each set of surface wears.
- After each complete set of abrasions are conducted (all control and test carriers abraded), the carriers are allowed to sit at least 15 minutes prior to being re-inoculated.
- The carriers are re-inoculated with 0.100 ml of the re-inoculation culture via spot inoculation, taking care to stay within 3mm of the edge of the test carrier and allowed to dry at ambient temperature for 10-20 minutes or until completely dry prior to initiation of the next set of abrasions.
- Cotton cloths used as part of wet abrasions are prepared individually prior to each wet abrasion cycle by spraying the cloth with sterile RO water using a sanitized Preval sprayer, from a distance of 75±1cm for no more than 1 second and used immediately.

| Hours | Minimum CFU/Carrier | Abrasion/ Re-inoculation Procedure |
|---|---|---|
| 0 | ≥1X10⁶ | Inoculation of All Carriers with initial inoculation culture |
| | | Test/Control Substance application and drying |
| | | Dry Abrasion (wear #1) |
| | | Re-inoculation (1)* |
| | | Wet Abrasion (wear #2) |
| | | Re-inoculation (2)* |
| | | Dry Abrasion (wear #3) |
| | | Re-inoculation (3)* |
| 1-48 | ≥1X10⁴ | Wet Abrasion (wear #4) |
| | | Re-inoculation (4)* |
| | | Dry Abrasion (wear #5) |
| | | Re-inoculation (5)* |
| | | Wet Abrasion (wear #6) |
| | | Re-inoculation (6)* |
| | | Dry Abrasion (wear #7) |
| | | Re-inoculation (7)* |
| | | Wet Abrasion (wear #8) |
| | | Re-inoculation (8)* |
| | | Dry Abrasion (wear #9) |
| | | Re-inoculation (9)* |
| | | Wet Abrasion (wear #10) |
| | | Re-inoculation (10)* |
| | | Dry Abrasion (wear #11) |
| | | Re-inoculation (11)* |
| | | Wet Abrasions (wear #12) |
| ≤48 | ≥1X10⁶ | Efficacy Test |

### Success Criteria:

- The experimental success (controls) criteria follow for Initial Reduction (Non-Continuous Claim):
   1. All media sterility controls must be negative for growth.
   2. Carrier contamination control must demonstrate negligible contamination.
   3. The media growth control must be positive for growth.
   4. All test microorganisms must demonstrate culture purity.
   5. Neutralization is validated as described previously.
   6. Soil sterility control is negative for growth.
   7. Re-inoculation Culture enumerations demonstrate ≥ 1 x 10⁴ CFU/carrier.
   8. Initial Numbers Control enumeration demonstrates ≥ 1 x 10⁶ CFU/carrier.
   9. Final (post contact time) Control Carrier count enumeration results demonstrate ≥ 1 x 10⁶ CFU/carrier.
- The experimental success (controls) criteria follow for Continuous Reduction:
   1. All media sterility controls must be negative for growth.
   2. Carrier contamination control must demonstrate negligible contamination.
   3. The media growth control must be positive for growth.
   4. All test microorganisms must demonstrate culture purity.
   5. Neutralization is validated as described.
   6. Soil sterility control is negative for growth.
   7. Initial inoculation control carriers must demonstrate an average ≥ 1 x 10⁶ CFU/carrier for a valid test.
   8. Re-inoculation control carriers must demonstrate an average ≥1 x 10⁴ CFU/carrier for a valid test.
   9. Final efficacy control carriers must demonstrate an average ≥1 x 10⁶ CFU/carrier for a valid test.
- Test substance performance criteria
   1. The results must show a bacterial reduction of at least 99.9% for treated carriers (laundered and unlaundered) and when compared to the parallel untreated control.

*** tend OF SPECIFATION OF THE PROTOCOL ***

The original EPA test method 90072PA4 was subsequently modified. The terms "EPA protocol 90072PA4 as modified" or "modified EPA protocol" as used herein refer to the original EPA test method 90072PA4 as specified above, with the following modifications:
*** START OF SPECIFATION OF THE MODIFICATION ***

Applicant seeking textile/fabric residual reduction (sanitization/disinfectant) claim must be tested for Residual reduction (sanitization/disinfectant) for multiple exposures (continuous reduction) at 4, 8, 12, 16, 18, and 24 hours after determined laundering cycles.
- Carriers must undergo a minimum of 11 re-inoculations for 24 hours claims (one for every 2 hours).
- A minimum of 4 hours with 4 hours increments (4, 8, 12, 16, 18, and 24) continuous bacterial reduction (or multiple bacterial exposure) claim is acceptable. Each 4 hours increment represent initial inoculation and one re-inoculation or two re-inoculations (see highlighted rows in table). These increments may be used as stopping time.
- Inoculation and re-inoculation should include at least 5% organic soil.
- Initial, re-inoculations, and final inoculations should yield a minimum CFU/carrier presented in the following table.

All re-inoculations are to be completed prior to the final efficacy evaluation test performed at least 24 hours after initial inoculation but not to exceed 48 hours.

| Hours* | Minimum CFU/Carrier | Inoculation/Re-inoculation Procedure | Log₁₀ Reduction |
|---|---|---|---|
| 0-1 | • 1X10⁶ for Bacteria and TB | Inoculation of All Carriers with initial inoculation culture | None |
| | • 1X10⁵ for Fungi | | |
| | • 1X10⁴ for Viruses | | |
| | | Re-inoculation (1) | |
| | | Re-inoculation (2) | |
| | | Re-inoculation (3) | |
| | • 1X10⁴ for Bacteria and TB | Re-inoculation (4) | |
| 1-24 | | Re-inoculation (5) | |
| | • 1X10³ for Fungi | Re-inoculation (6) | None |
| | • 1X10² for Viruses | Re-inoculation (7) | |
| | | Re-inoculation (8) | |
| | | Re-inoculation (9) | |
| | | Re-inoculation (10) | |
| | | Re-inoculation (11) | |
| 24 | 1X10⁶ for Bacteria | Residual Bacterial Reduction | 3 log₁₀ reduction for bacterial |
| | 1X10⁶ for Bacteria | Residual Self-Sanitization Test | 3 log₁₀ reduction for bacterial |
| | • 1X10⁶ for Bacteria and TB | Residual Self-Disinfection Test | • 5 log₁₀ reduction for Bacteria and TB |
| | • 1X10⁵ for Fungi | | • 4 log₁₀ reduction for Fungi |
| | • 1X10⁴ for Viruses | | • 3 log₁₀ reduction for Viruses |

*** END OF SPECIFATION OF THE MODIFICATION ***

### Working Examples

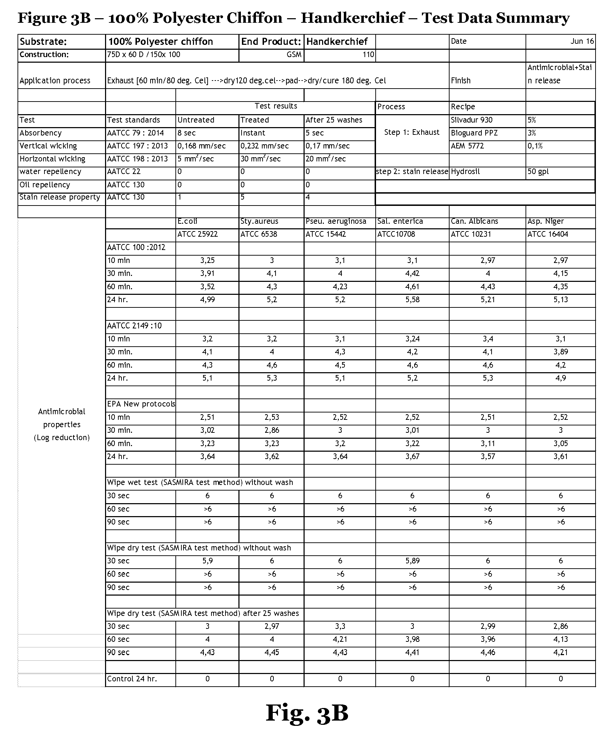

Fig. 3A and 3B summarize the starting textile materials, the finishing processes and recipes used to produce two different Working Examples according to preferred embodiments of the invention, one based on cotton and one based on polyester.

### Working Example 1

Working Example 1 will now be described with reference to the table in Fig. 3A.

The starting textile of the Working Example 1 was a 100% cotton poplin (or popline), a strong and tightly woven fabric, with count 40s warp and 40s weft, construction 144 x 98, width 150 cm, fabric weight 135 g/m². Such a fabric may be used, for instance, for handkerchiefs.

In a first process cycle, the fabric was exhausted and then dried. In a second process cycle, the fabric was padded, then dried again and cured.

In the exhaustion process, 300 kg (about 1,500 meters) of starting textile material were loaded on a jigger machine (Yamuna, model number Y1100). Into about 600 liters of water (material to liquor ratio of about 1:2), 9 kg (3% of the weight of the textile material) of Texguard 20 were added, resulting in an amount of 0.6% PHMB o.w.f., furthermore 3 kg (1% of the weight of the textile material) of Silvadur 930, resulting in an amount of 0.0017% silver o.w.f., 9 kg (3% of the weight of the textile material) of Biogard PPZ, resulting in an amount of 0.75% propiconazole o.w.f. The pH of the liquor was adjusted with 0.03 gpl of citric acid and maintained between pH 5 and pH 6, preferably at pH 5.5. The temperature of the liquor was set to 80 °C.

The jigger machine was started and run at a speed of 50 m/s, and the run was continued for the next 60 minutes (2 ends, with a break of less than 30 seconds between the ends). The liquor was constantly stirred with a stirrer at a speed of 300 rpm throughout the exhaustion process. The exhaustion rate was about 98%. Following this, the process bath was drained and the textile material was immediately transported to a stenter machine for drying. Thus, the exhaust time was 60 minutes.

The textile was dried by passing it through the stenter, which had 8 chambers and a length of 24 meters, at a speed of 20 centimeters per second. The maximum temperature of 120 °C was applied in all 8 chambers, i.e. during 120 seconds.

Following this first process cycle, a second process cycle with a padding process was performed to apply a hydrophilic/stain release agent: To about 400 liters of water, 20 kg (50 gpl) of Hydrosil were added, resulting in a concentration of 15 gpl organosilane terpolymer in the liquor. During the padding process, the liquor had a temperature of 30 °C. The padding mangle pressure was 2 bar. The nominal pick-up rate was 100%. Estimating that as mentioned above, after the first process cycle, the effective pick-up rate in a padding process of subsequent process cycles is about 70% of the nominal pick-up rate, the amount of organosilane terpolymer adhered to the textile material was about 1.05% o.w.f.

The textile material was then dried and cured for in total 2 minutes in a single pass through the stenter, at a maximum temperature of 180 °C. The maximum curing temperature was applied for 60 seconds (in 4 of the 8 chambers of the stenter).

Different tests regarding hydrophilicity, stain release capability, and antimicrobial efficiency were performed on the untreated starting textile material and on the finished textile of Working Example 1, the results of which are summarized in the table of Fig. 3A.

With regard to hydrophilicity, the treated fabric exhibits essentially instant water absorbency, while the untreated fabric exhibits a water absorbency time of 3 seconds. Both were measured in accordance with AATCC test method 79-2014 (Option A). The vertical wicking rate of the treated textile increased only slightly, from 0.64 to 0.7 mm/s, measured in accordance with AATCC test method 197-2013. However, the horizontal wicking rate increased significantly from 6.05 to 59 mm²/s, measured in accordance with AATCC test method 198-2013. The water repellency rating measured in accordance with AATCC test method 22-2014 was 0 both before and after finishing the textile. After 25 laundry washes (washes as described above), the hydrophilicity had slightly decreased. For example, the horizontal wicking rate had decreased to 25 mm²/s. However, water absorbency time was still instantaneous. The wash-durability of the increased hydrophilicity imparted by the finishing process is therefore satisfactory.

Both the starting textile material and the finished textile had an oil repellency rating of grade 0 when measured in accordance with AATCC test method 118-2013, which means that both absorb oily stains well. However, the finishing of the textile had the effect that the stain release capability increased from the lowest grade (1) to the highest grade (5) as measured in accordance with AATCC test method 130-2010. After 25 laundry washes, the stain release capability had slightly decreased, to grade 4. The wash-durability of the improved stain release properties imparted by the finishing process is therefore satisfactory.

While the starting textile material did not exhibit any measurable antimicrobial properties, the finished textiles were highly antimicrobial, and the imparted antimicrobial properties proved to be sufficiently wash-durable.

The results following the AATCC 100-2012 and the ASTM 2149-10 test methods of the unwashed textiles showed that the treated fabric is capable of reducing all of the above-mentioned organisms by more than 99,9% (3 log) within 10 minutes of contact time. With increasing contact time (up to 24 hours), microbial reduction values increased up to more than 99,999% (5 log) for most microbial cultures. The treated unwashed fabric reduced all microbes on a ceramic surface after 30/60/90 seconds of wiping by 99,9999/>99,9999/>99,9999% (6/>6/>6 log) as evaluated 60 minutes post wiping, according to the SASMIRA wiping test method described above when the wipe was wetted. The same test performed with a dry wipe showed almost the same results.

Wash-durability of the antimicrobial properties where, first of all, shown with the modified EPA protocol. As explained above, the modified EPA protocol assesses, based on AATCC test method 100-2012, the efficiency of antimicrobial finishes of textiles which were washed in 25 laundry cycles and abraded in 12 abrasion cycles. Fig. 3A shows that the washed on abraded finished textile still exhibits microbial reduction values of more than 2.5 log for all organisms within 10 minutes of contact time, and more than 3.8 log within 24 hours of contact time. Also the SASMIRA wiping test (dry wipes) was performed with a finished textile that had been laundered 25 times. Reduction values on the surface after 30/60/90 seconds of wiping were about 3/4/4.5 log) for almost all organisms.

### Working Example 2

Working Example 2 will now be described with reference to the table of Fig. 3B.

The starting textile of Working Example 2 was a 100% polyester chiffon, a lightweight woven fabric with construction 75D x 60D / 150 x 100, width 150 cm, fabric weight 110 g/m². Like the fabric of Working Example 1, such a fabric may be used, for instance, for handkerchiefs.

The overall process used for finishing Working Example 2 was the same as the process for finishing Working Example 2. However, a different recipe was used in the antimicrobial exhaust process. The liquor was prepared such that it contained Silvadur 930 in an amount of 5% o.w.f., Biogard PPZ in an amount of 3% o.w.f., and AEM 5772 in an amount of 0.1% o.w.f. This results in silver being applied to the textile material in an amount of 0.0085% o.w.f., propiconazole in an amount of 0.75% o.w.f., and quaternary ammonium organosilane in an amount of 0.072% o.w.f. The concentration of Hydrosil in the liquor of the padding process was the same as for Working Example 1 (50 gpl).

Since polyester is somewhat hydrophilic in nature, the water absorbency time of the starting textile material was 8 seconds. In the unwashed finished textile, the absorbency time was reduced to instantaneous. After 25 laundry washes, absorbency time increased back to 5 seconds. The finishing process increased the horizontal wicking rate from 5 mm²/sec to 30 mm²/sec. After 25 laundry washes, absorbency time had decreased back to 20 mm²/sec.

As in Working Example 1, the finishing process increased the stain release rating of the textile of Working Example 2 from grade 1 to grade 5. After 25 laundry washes, the rating had slightly gone down to grade 4.

The antimicrobial efficiency of Working Example 2 was essentially the same as that of Working Example 1, both for unwashed samples and after 25 laundry washes.

### Experimental Examples

The inventor of the present invention performed comprehensive further experiments to determine the effect of different recipes and finishing process parameters. The finishing processes were in general as described above in the context of the Working Examples. Where differences exist, these will be discussed below. Most Experimental Examples were produced with the two different starting textile materials (100% cotton and 100% polyester) described above in the context of the Working Examples.

### Experimental Examples 1: Different combinations of antimicrobial agents

With Experimental Examples 1, the effect of the application of different numbers of agents to the starting textile materials was studied. The agents applied to the cotton material were taken from the group of silver, PHMB, and propiconazole, which are the three most preferred antimicrobial agents for treating cellulosic textile materials. The agents applied to the polyester material were taken from the group of silver, propiconazole, and quaternary ammonium organosilane compounds, which are the three most preferred antimicrobial agents for treating synthetic textile materials.

The textiles were treated with (1) each of the agents from the respective group individually, (2) all three tuple combinations of agents from the respective groups, and (3) all three agents from the respective groups together. The total amount of the chemicals in all experiments was 6% o.w.f. Thus, where one single agent was applied to the textile, 6% o.w.f. of the respective chemical was used, where a combination of two agents was applied, 3% o.w.f. of each of the respective two chemicals was used, and where a combination of three agents was applied, 2% o.w.f. of each of the respective three chemicals was used.

The finishing process was as described above in the context of the working examples, but since no hydrophilic/stain release agent was applied, there was only one process cycle. The fabric was exhausted and then dried and cured in one single pass through the stenter, with the process parameters as described above.

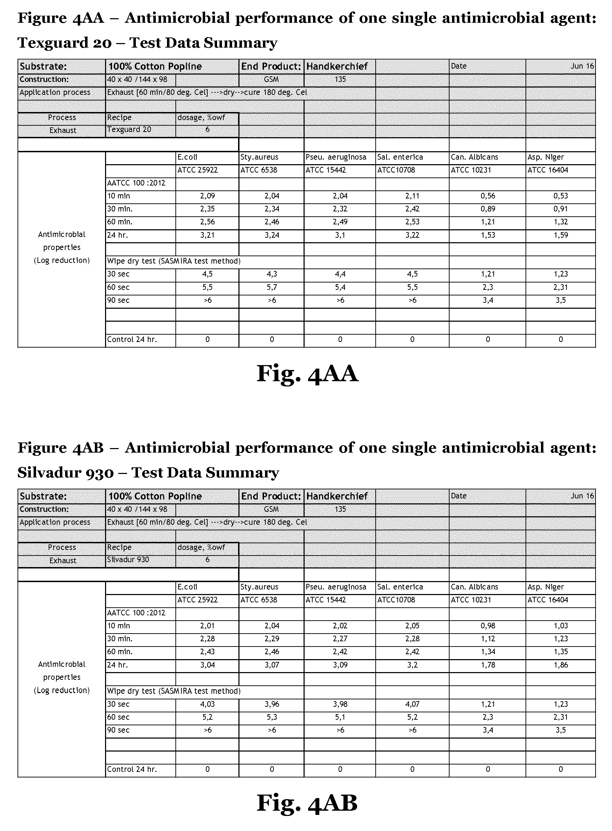

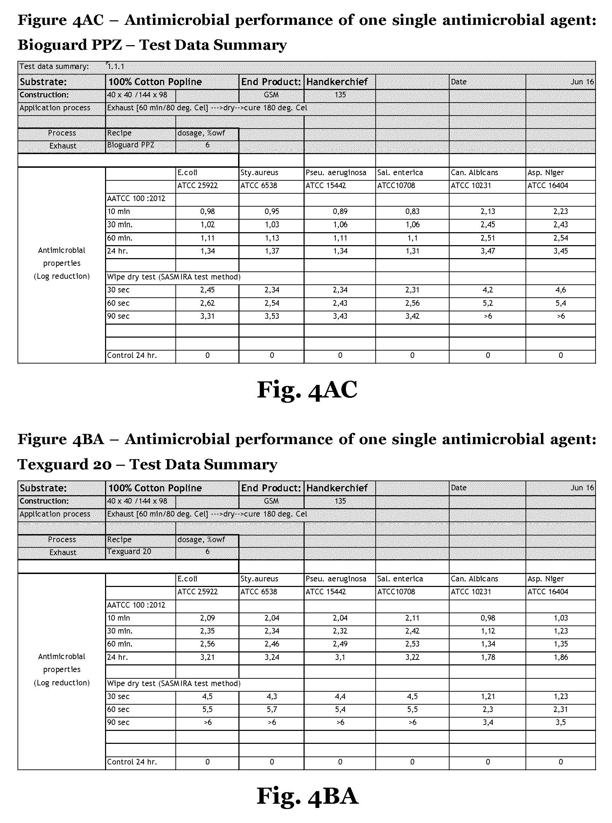

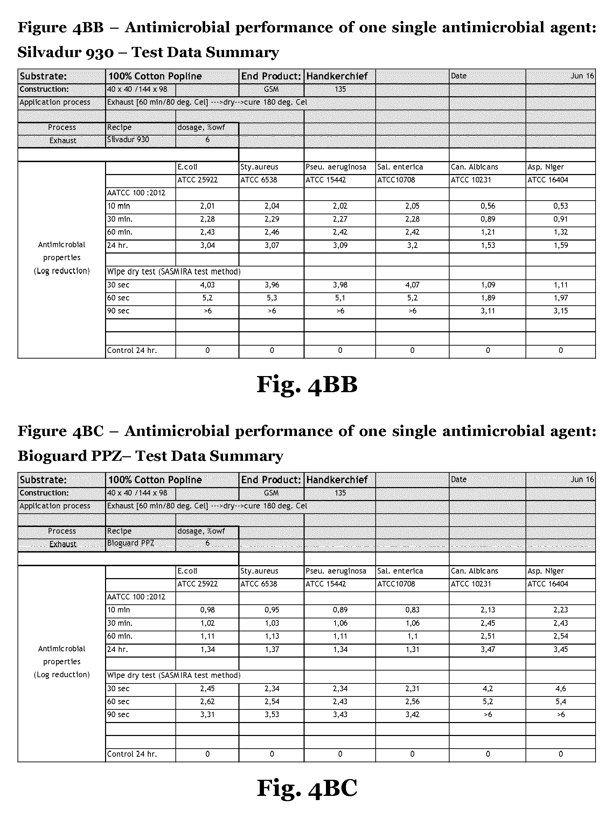

Fig. 4AA to 4AC show the antimicrobial performance of the cotton textiles treated with one single agent. The results indicate that PHMB and silver applied individually are about equally efficient for killing bacteria (*Escherichia coli*, *Staphylococcus aureus, Pseudomonas aeruginosa,* and *Salmonella enterica*). However, they perform poorly when it comes to killing fungi (*Candida albicans* and *Aspergillus Niger).* On the other hand, propiconazole is efficient for killing fungi, but not efficient for killing bacteria.

Fig. 4BA to 4BC show the antimicrobial performance of the polyester textiles treated with one single agent. Similar to the results for silver and PHMB on cotton, the results for polyester indicate that silver and quaternary ammonium organosilane compounds applied individually are about equally efficient in killing bacteria, but not efficient for killing fungi. However, unlike propiconazole adhered to cotton, propiconazole adhered to polyester is about as efficient against bacteria as silver and quaternary ammonium organosilane adhered to polyester. Furthermore, like propiconazole adhered to cotton, propiconazole adhered to polyester works well against fungi. The overall killing rates of the treated cotton materials were about the same as the killing rates of the treated polyester materials.

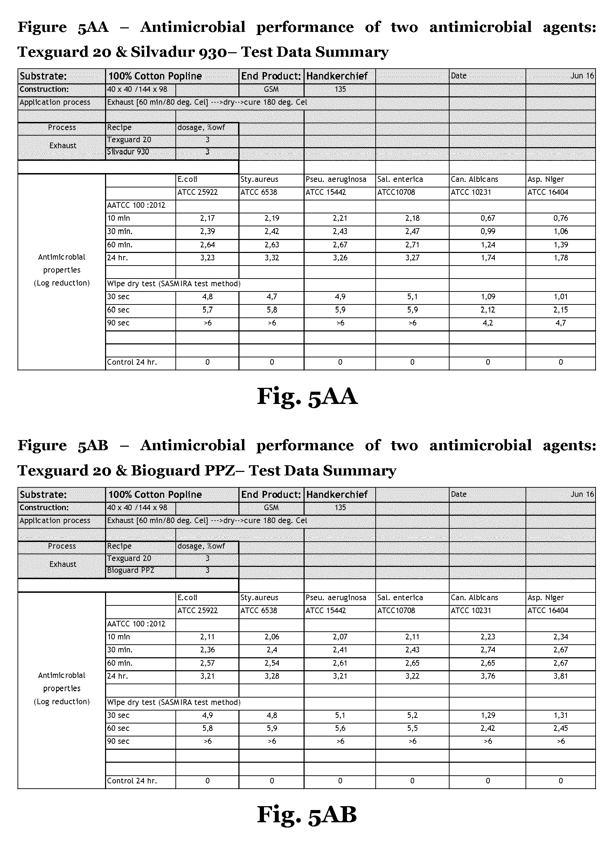

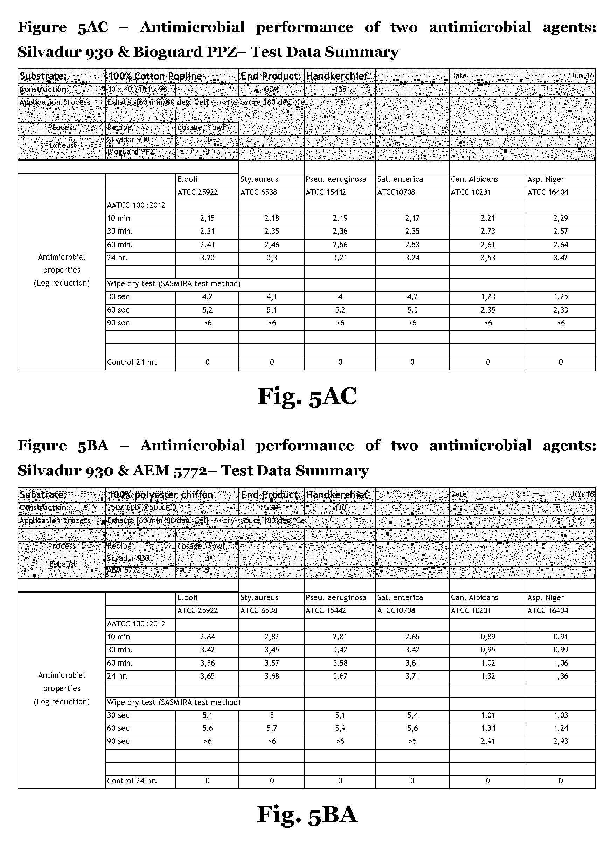

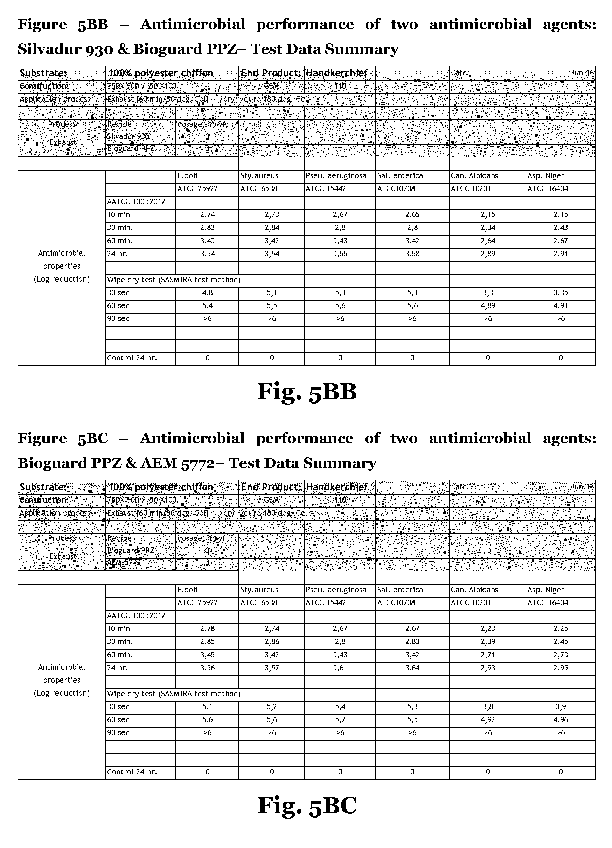

Fig. 5AA to 5AC show the antimicrobial performance of the cotton textiles treated with combinations of two agents. The textile which was not treated with propiconazole (i.e. the textile treated with silver and PHMB) performed poorly against fungi. On the other hand, the textiles treated with propiconazole and either silver or PHMB performed well against both bacteria and fungi. The overall performance of the textiles treated with combinations of two agents was slightly higher than the performance of the textiles treated with one single agent.

Fig. 5BA to 5BC show the antimicrobial performance of the polyester textiles treated with combinations of two agents. Again, the textile which was not treated with propiconazole (i.e. the textile treated with silver and quaternary ammonium organosilane compounds) performed poorly against fungi. On the other hand, the textiles treated with propiconazole and either silver or quaternary ammonium organosilane compounds performed well against both bacteria and fungi. The overall performance of the textiles treated with combinations of two agents also here was slightly higher than the performance of the textiles treated with one single agent.

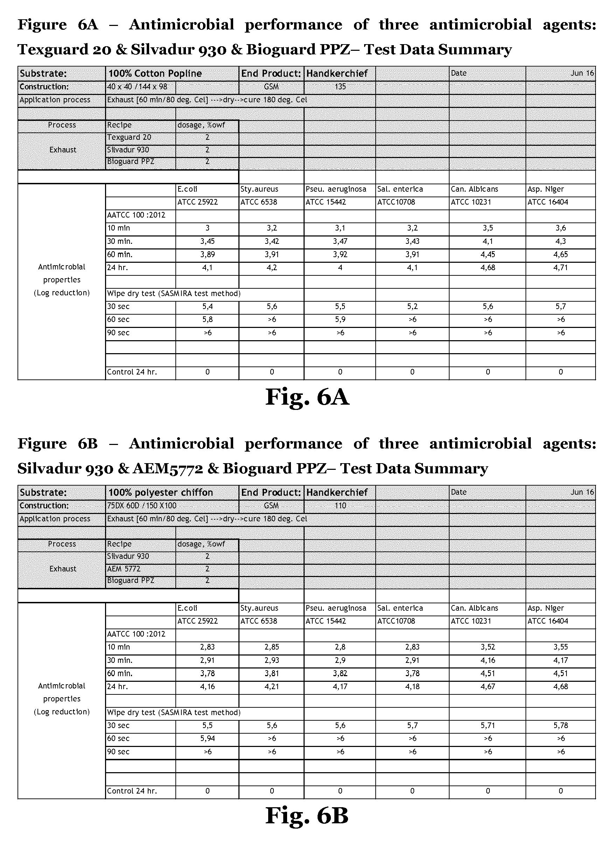

Fig. 6A and 6B show the antimicrobial performance of the cotton and polyester textiles, respectively, treated with combinations of all three agents from the above-specified groups. Overall performance is again higher than the performance of the textiles treated only with two agents. This seems to make a difference in particular for the shorter wiping periods (30 seconds), where the achievable killing rates on the surface are about 4.5 log for one single agent, about 5 log for a combination of two agents, and about 5.5 log for a combination of three agents, for both cotton and polyester.

From Experimental Examples 1, it can be concluded that all antimicrobial agents identified as preferred by the inventor are about equally powerful against bacteria. This was also found in earlier experiments conducted by the inventor and discussed in International patent application PCT/EP2016/054245 by the same applicant. An exception to this rule is propiconazole (or other azole-based agents), which performs poorly against bacteria when adhered to cotton (or other cellulosic textile materials). The inventor believes that propiconazole gets more efficiently attached to synthetic textiles like polyester than to cellulosic textiles like cotton because polyester is thermoplastic polymer. Furthermore, only propiconazole (or other azole-based agents) or combinations comprising propiconazole show a good performance against fungi. Cotton (or other cellulosic textile materials) and polyester (or other synthetic textile materials) are generally equally suitable unless only propiconazole (or another azole-based agent) is applied, in which case polyester performs better. The experiments show a synergistic killing effect for the same organism with a growing number of agents. Although the effect is not dramatic, it is well visible.

### Experimental Examples 2: Effect of hydrophilic/stain release agents

The purpose of Experimental Examples 2 was to study the effect of the hydrophilic/stain release agents, in particular in combination with antimicrobial agents.

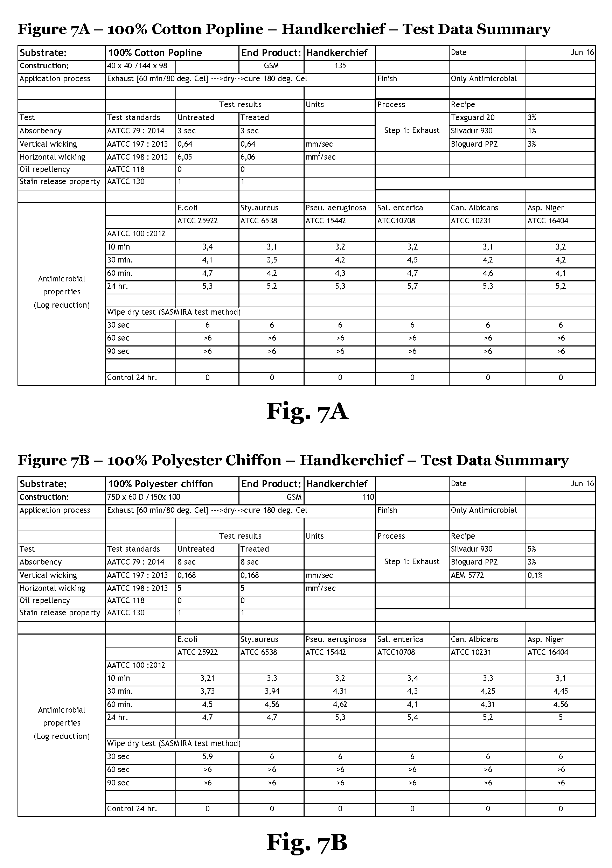

Fig. 7A and 7B show the data of Experimental Examples 2a, where only antimicrobial agents were applied to the textile materials. The finishing process was the same as for Experimental Examples 1, but the antimicrobial recipes were the preferred recipes for cotton and polyester, respectively, as used already in the Working Examples described above.

As can be seen in Fig. 7A and 7B, the hydrophilic/hydrophobic properties of the starting textile materials were not affected by the treatment with antimicrobial agents. The same is true for the stain release rating. It can therefore be concluded that any change in hydrophilicity and stain release rating observed above for the Working Examples can be attributed to the treatment with Hydrosil in the second process cycle, which contains the hydrophilic/stain release agent organosilane terpolymer.

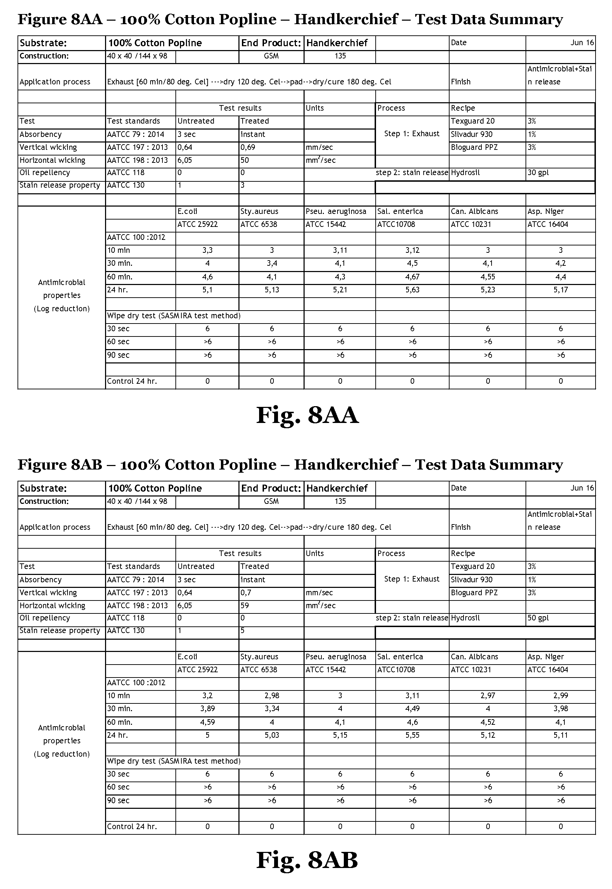

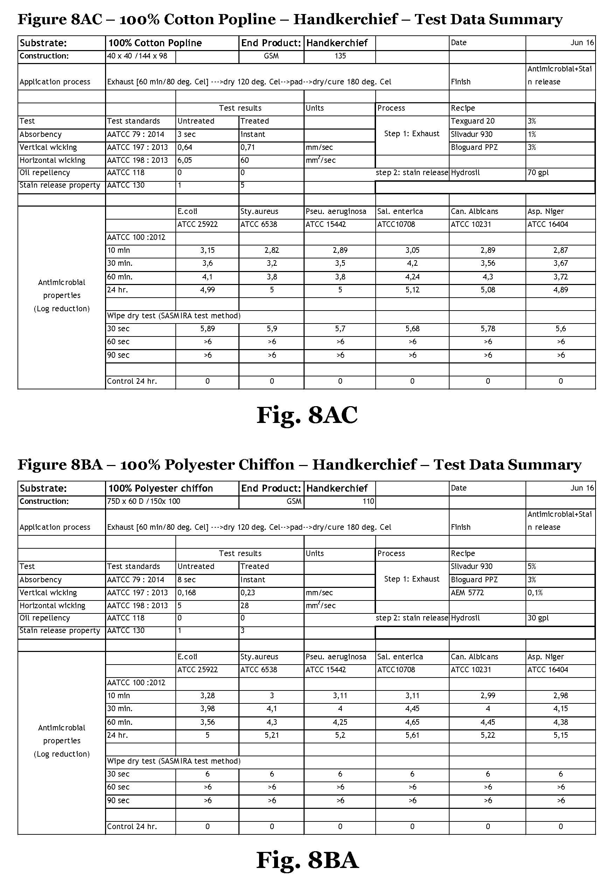

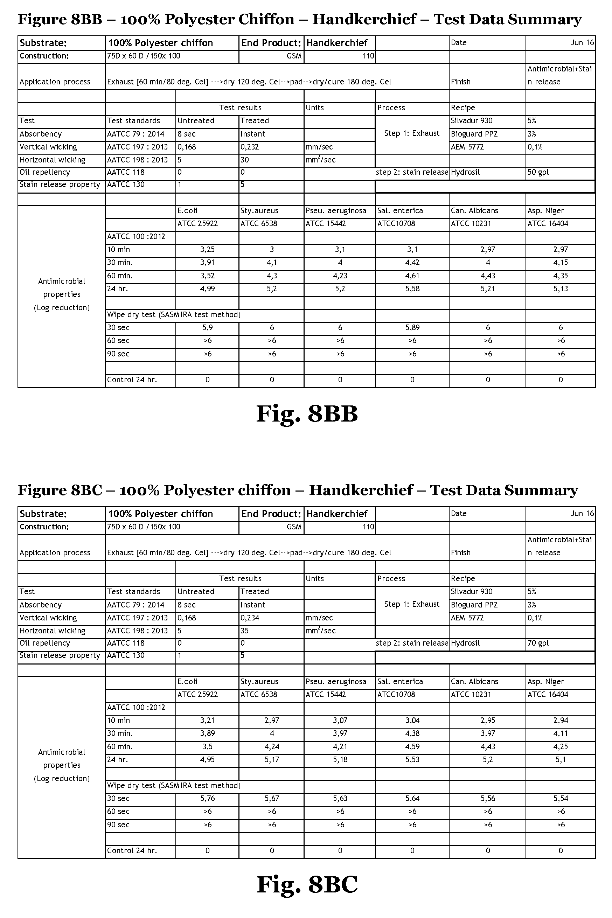

Fig. 8AA to 8AC (cotton) and Fig. 8BA to 8BC (polyester) show the data of the textile materials of Experimental Examples 2b, which were treated with the same antimicrobial process as the Experimental Examples 2a of Fig. 7A and 7B. However, these textiles were treated with Hydrosil in a second process cycle as described in the context of the Working Examples. Varying amounts of Hydrosil were added to the liquor of the padding process: 30 gpl (Fig. 8AA and 8BA), 50 gpl (like in the Working Examples, Fig. 8AB and 8BB), and 70 gpl (Fig. 8AC and 8BC).

The test results show that Hydrosil improves absorbency time and horizontal wicking significantly, as has already been observed in the context of the Working Examples. Vertical wicking is only marginally (cotton) or slightly (polyester) improved. Between an application of 30 gpl of Hydrosil in the padding liquor and 50 gpl, the stain release rating improves from grade 3 to grade 5. No significant changes can be observed in hydrophilicity and stain release capability when increasing the Hydrosil concentration in the liquor from 50 gpl to 70 gpl. The antimicrobial efficiency decreases slightly with increasing amounts of Hydrosil.

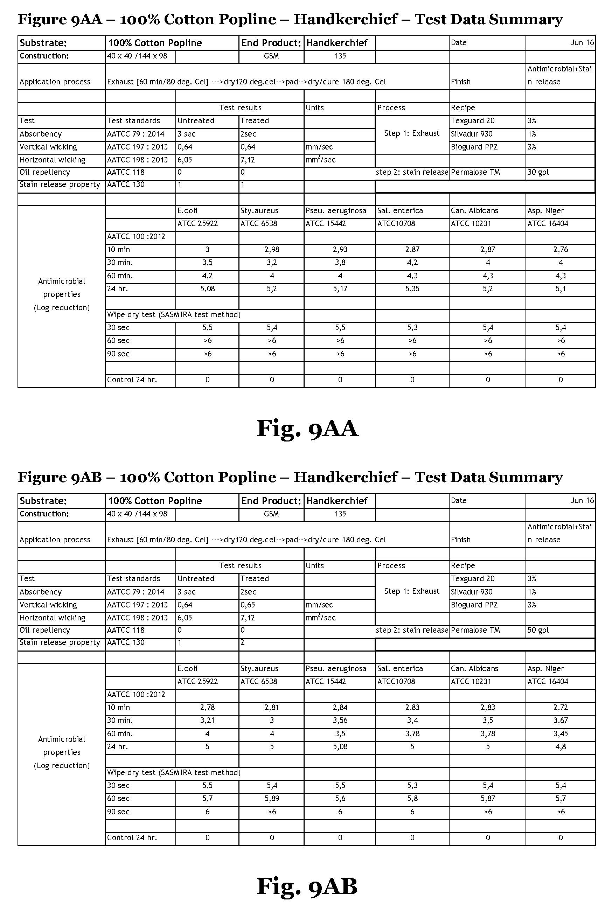

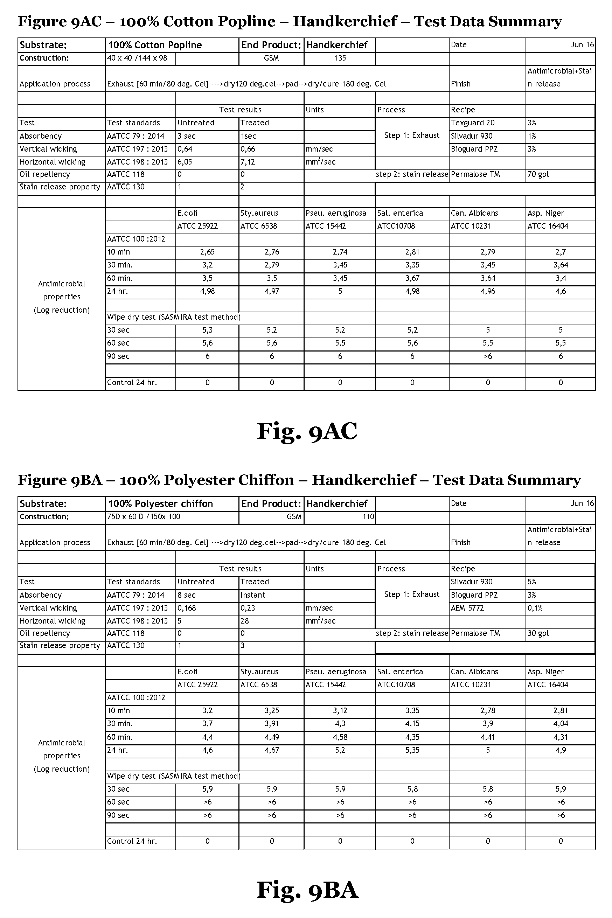

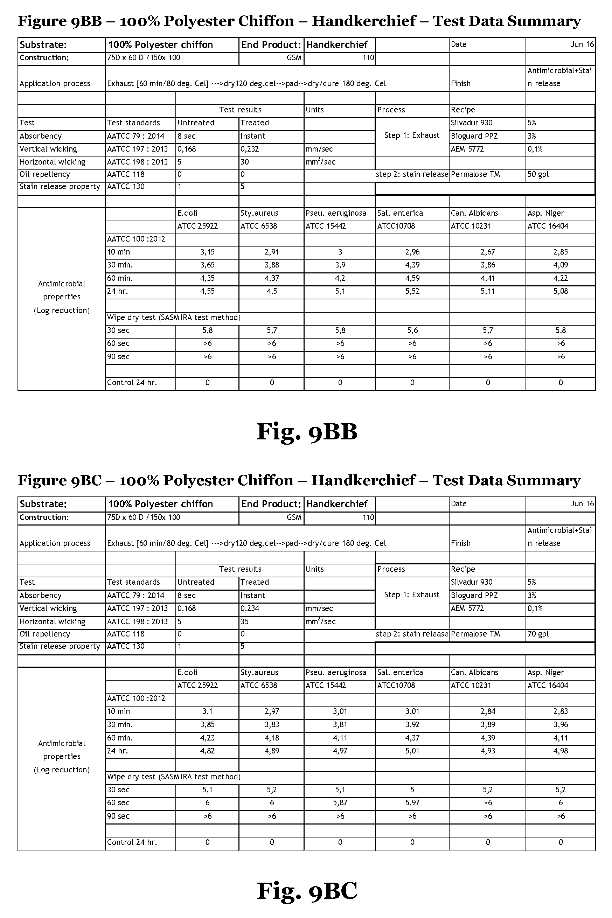

Fig. 9AA to 9AC (cotton) and Fig. 9BA to 9BC (polyester) show the data of the textile materials of Experimental Examples 2c, which were treated with the same antimicrobial process as the Experimental Examples 2a and 2b. However, in the second process cycle, Permalose was applied in the padding process of the second process cycle instead of Hydrosil.

The treatment with Permalose increases the hydrophilic properties of cotton only slightly, which is not unexpected given that it is optimized for synthetic materials. Also the stain release rating of cotton is only improved to grade 2 (for 50 and 70 gpl). The antimicrobial efficiency decreases a little faster with increasing amounts of Permalose than with increasing amounts of Hydrosil. On the other hand, on polyester (Fig. 9BA to 9BC), Permalose improves hydrophilicity and stain release capability equally well as Hydrosil, but again the antimicrobial efficiency decreases a little faster with increasing amounts of Permalose than with increasing amounts of Hydrosil.

From Experimental Examples 2, it can be concluded that it is not the antimicrobial agents used herein but only the hydrophilic/stain release agents which affect hydrophilicity and stain release capability. Furthermore, Permalose is preferred only for polyester or other synthetic textile materials. The optimum concentration of both Hydrosil and Permalose in a padding process with a pick-up rate of 100% seems to be 50 gpl, which results in actives adhered to the textile material in an amount of about 1% o.w.f. (organosilane terpolymer/Hydrosil) and about 0.4% o.w.f. (fatty alcohol ethoxylates/Permalose).

As mentioned above, a chemical which is equivalent to Permalose but optimized for cellulosic textiles is Hydroperm of Archroma. It comprises 20% of fatty alcohol ethoxylates. The inventor found in experiments, whose results are not reproduced herein, that also for Hydroperm, a concentration of 50 gpl in the padding liquor brings about the best results at a pick-up rate of 100%. With an estimated effective pick-up rate of 70%, the amount of fatty alcohol ethoxylates adhered to the textile is then about 0.7% o.w.f

### Experimental Examples 3: Variations in the finishing process

In previous research efforts related to processes of conveying antimicrobial properties to textiles, the inventor had already found out that for the exhaustion process, a temperature of the exhaust liquor of 80 °C and an exhaust time of 60 min is ideal in terms of antimicrobial efficiency and wash-durability thereof, see International patent application PCT/EP2016/054245 by the same applicant. Furthermore, application of an exhaustion process with these parameters leads to higher antimicrobial efficiency and wash-durability than application of a padding process. Finally, the ideal curing temperature had been determined to be 180 °C. Experimental Examples 3 were manufactured to confirm that these earlier findings hold true also when a hydrophilic/stain release agent is applied to the textile, subsequent to the application of the antimicrobial agents.

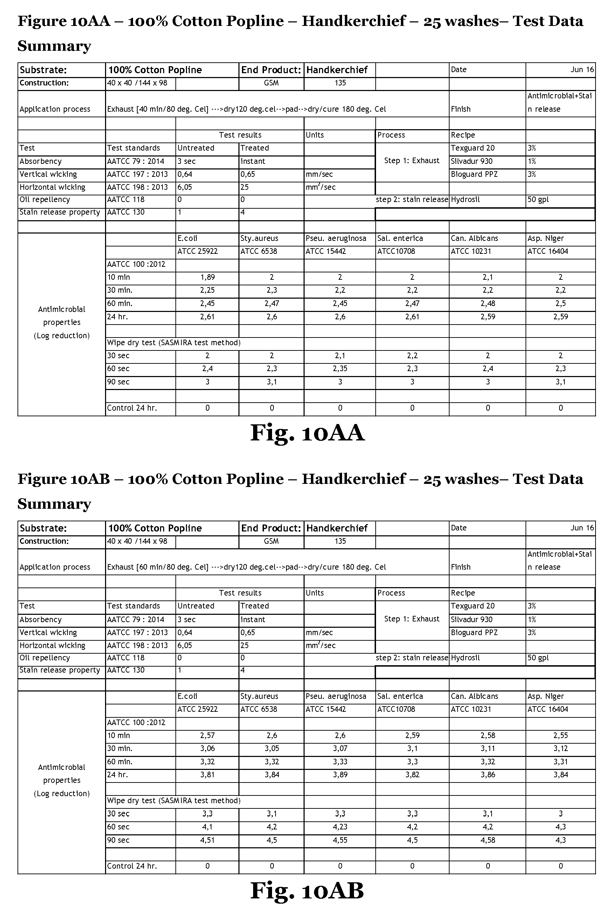

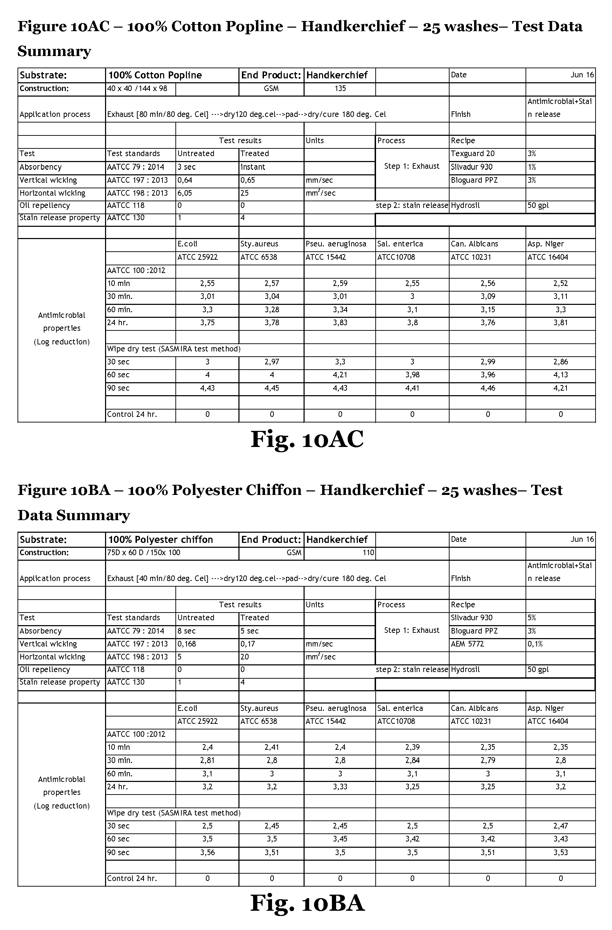

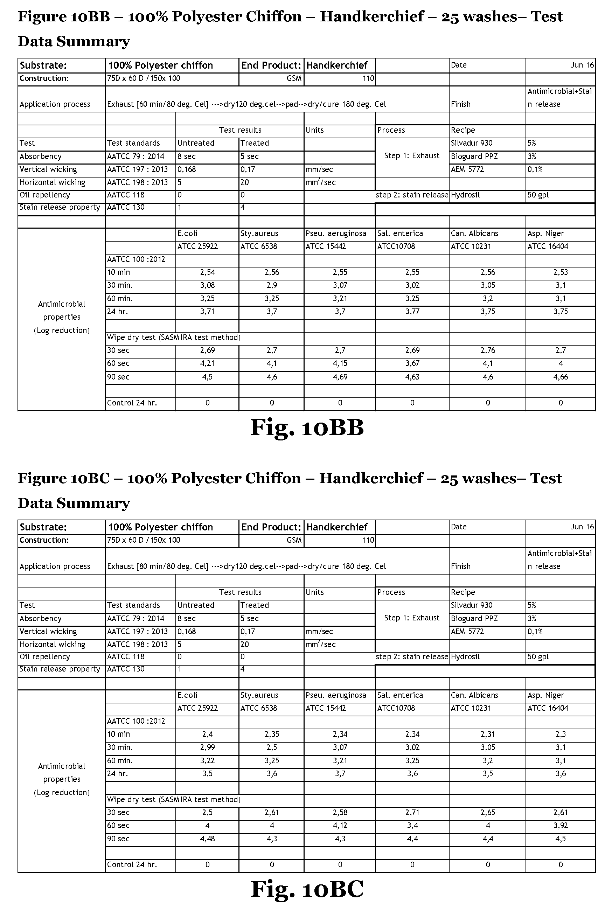

Fig. 10AA to 10AC (cotton) and Fig. 10BA to 10BC (polyester) show the data of the textile materials of Experimental Examples 3a. They were manufactured by finishing the starting textile material with the same antimicrobial exhaustion process cycle followed by a hydrophilic/stain release padding process cycle, and with the same recipes, as described above for the Working Examples. However, the exhaust time was varied between 40/60/80 minutes.

The performance tests were made on the finished textile after 25 laundry washes. As could be expected, the variation in exhaust time in the antimicrobial process cycle had no impact on the hydrophilic and stain release properties. However, the tests confirmed that the antimicrobial performance after 25 washes for the samples treated with an exhaust time of 60 minutes is considerably higher (about 1 log) than that of the samples treated for 40 minutes, and slightly higher than that of the samples treated for 80 minutes.

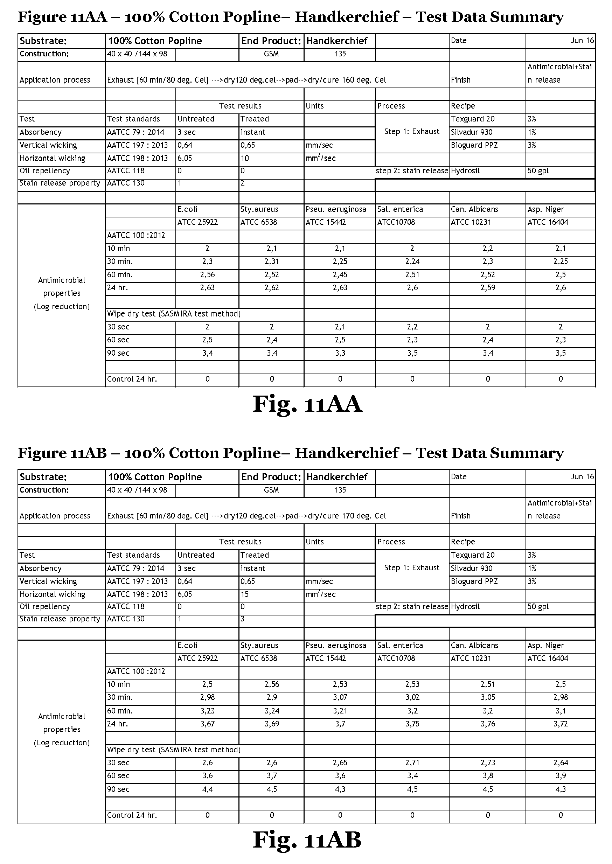

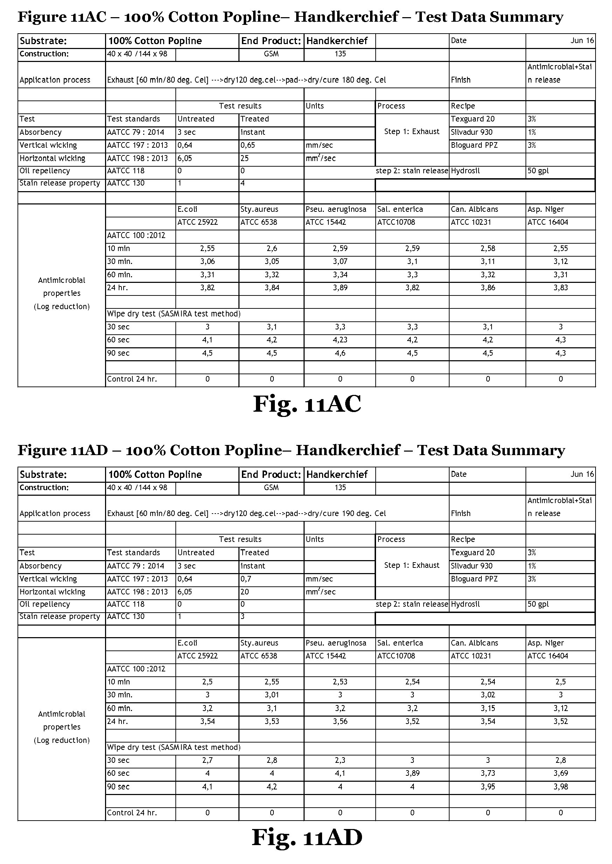

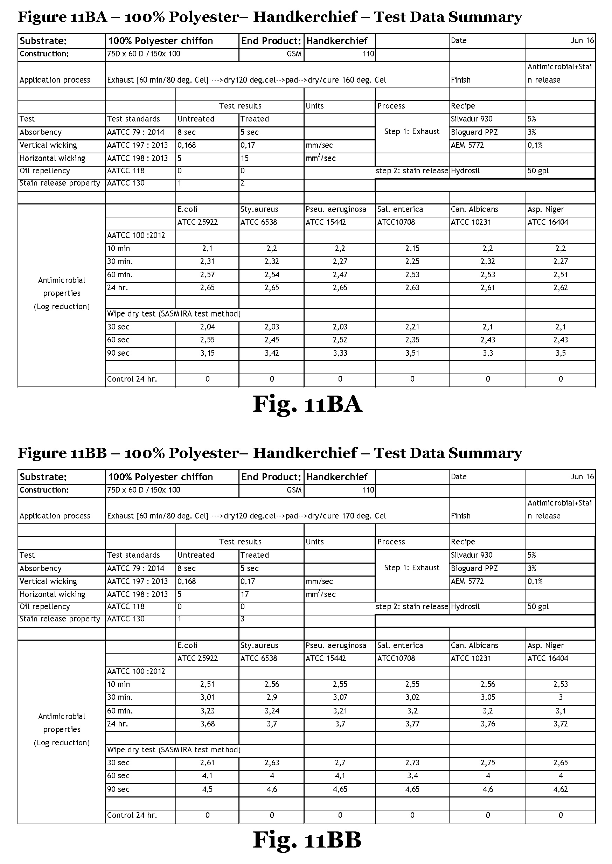

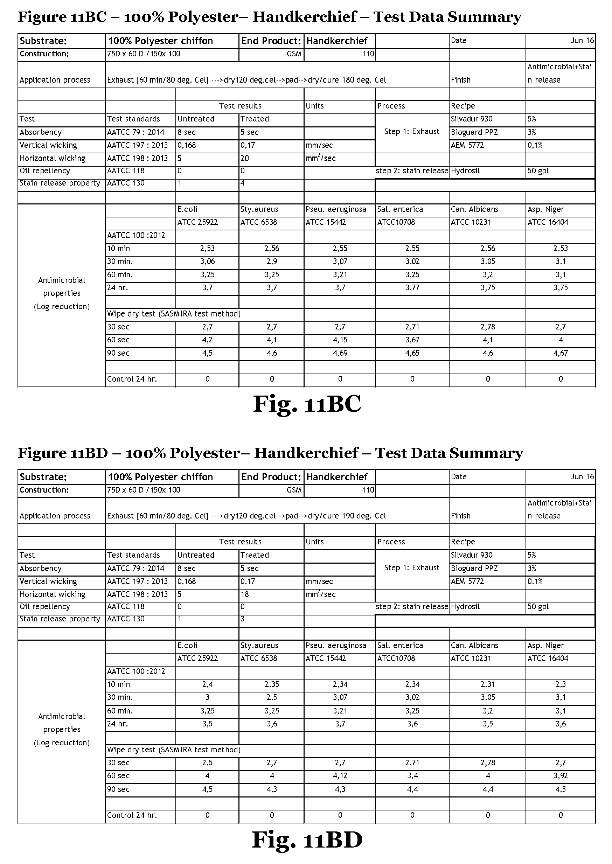

Fig. 11AA to 11AD (cotton) and Fig. 11BA to 11BD (polyester) show the data of the textile materials of Experimental Examples 3b. They were finished with the same process as described above for the Working Examples, but the curing time (at the end of the second process cycle) was varied between 160/170/180/190 minutes.

The performance tests were again made on the finished textile after 25 laundry washes. The hydrophilic properties peaked for a curing temperature of 180 °C. This could be observed in the horizontal wicking rate, which increased for the cotton textile material from 10 mm²/sec (curing temperature of 160 °C) to 25 mm²/sec (curing temperature of 180 °C), and then declined again to 20 mm²/sec (curing temperature of 190 °C). Similar results could be observed for the polyester textile material. However, the absorption time was the same for all curing temperatures, namely instantaneous for cotton and 5 seconds for polyester.

A similar observation could be made for the stain release capability, which for both cotton and polyester peaked at grade 4 for the curing temperature of 180 °C. For the lower curing temperatures of 160 °C and 170 °C, the stain release ratings were grade 2 and grade 3, respectively. For the curing temperatures of 190 °C, the stain release rating started to decline again, to grade 3.

Finally, also the antimicrobial performance peaked for both cotton and polyester at the curing temperature of 180 °C. A considerable performance increase could be observed when the curing temperatures went from 160 to 170 °C, and a slight increase when the curing temperature went from 170 to 180 °C. When the curing temperature was further increased to 190 °C, the performance started to decline again.

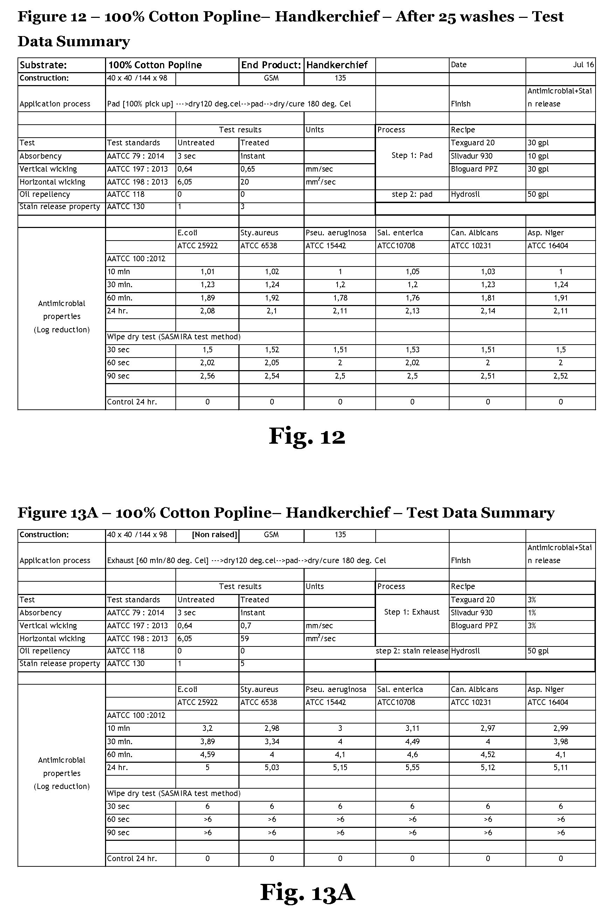

Fig. 12 shows the data of the textile material of Experimental Example 3c, which was finished using a padding process in the first (the antimicrobial) cycle, instead of an exhaust process. The concentrations of the antimicrobial chemicals in the padding liquor were chosen such that the amount of antimicrobial agents adhered to the finished textile material were the same as for the examples finished with an exhaust process. This experiment was only made with cotton, not with polyester.

The performance tests were again made on the finished textile after 25 laundry washes. Interestingly, hydrophilicity and the stain release capability were somewhat lower than in the examples finished with an exhaust process in the antimicrobial cycle. For example, the vertical wicking rate was only 20 mm²/sec instead of 25, and the stain release rating was only grade 3 instead of grade 4. It is believed that if the antimicrobial agents are padded in the first cycle, they occupy more space on the fiber surface than when they are exhausted, leaving less space for the hydrophilic/stain release agents to be adhered in the padding process of the subsequent cycle.

More importantly, the antimicrobial performance of Experimental Example 3c was much lower (by about 2 log) than that of the examples where an exhaustion process was used for applying the antimicrobial agents.

From Experimental Example 3c, it can be concluded that applying the antimicrobials with an exhaustion process leads to much better antimicrobial performance and/or wash-durability of the antimicrobial properties than the application of a padding process. It even increases the hydrophilic and stain release properties conveyed in a subsequent padding process, and/or the wash-durability thereof. Experimental Example 3a confirmed that even when hydrophilic/stain release agents are applied to a textile which had been treated with antimicrobials in an exhaust process, an exhaust time of 60 minutes is ideal for antimicrobial performance and/or wash-durability thereof. Finally, Experimental Example 3b showed that a curing time of 180 °C is ideal not only for antimicrobial performance but also for hydrophilic and stain release capability, and/or wash-durability thereof.

### Experimental Examples 4: Non-raised versus peached versus looped fabric

As was mentioned above, the starting textile material is advantageously a raised fabric, such as a peached or looped fabric. Experimental Examples 4 were manufactured and tested in order to find out whether these properties have an influence on hydrophilic, stain release, and in particular on antimicrobial performance with regard to surface sanitizing.

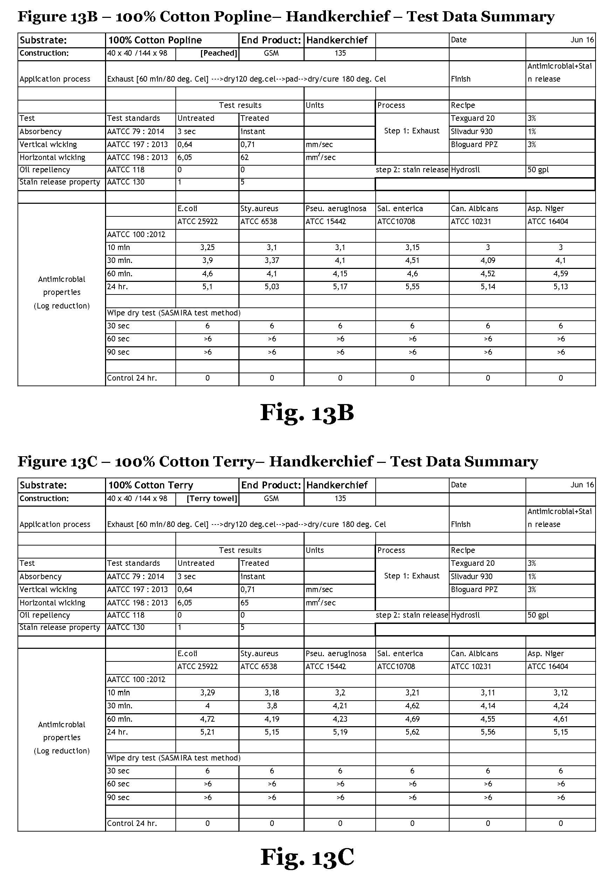

As can be seen in Fig. 13A to 13C, three different starting cotton textile materials were finished: (1) the peached cotton popline as it was used in the examples above, (2) the same material non-raised, and (3) a terry towel with otherwise equal properties, namely count 40s warp and 40s weft, construction 144 x 98, fabric weight 135 g/m².

The test results shown in Fig. 13A to 13C seem to suggest that there is substantially no difference in hydrophilic/stain release/antimicrobial performance between the three different textiles, not even with regard to the capability of sanitizing a surface.

### Examples for the use of the finished textile material

Embodiments of the textile materials according to the invention can be used, e.g., as a handkerchief, a towel, in particular a face, kitchen, hospital, or a baby towel, a floor mob, or a duster, in particular table duster, or similar hygiene product. Aqueous, oily, or other liquids, in particular body liquids like sweat or blood, food rests, dust, stains, or other dirt or dirt particles can be removed from a surface and/or the surface can be cleaned of such materials, by wiping the textile material over the surface, whereby the surface may be cleaned and/or dried. Examples for such surfaces are a floor, a kitchen top, a table, the surface of a medical device, a surface of furniture in a doctor's office or in a hospital, in particular in an operating theater or emergency room, the surface of a door handle, or the surface of the housing of an electrical consumer product. The surface may also be living human or animal skin, preferably skin of the human hand or face. The textile material may be used dry or wetted, and it may be used with cleaning agents.

Where such a hygiene product was treated with hydrophilic and/or stain release agents, usability and/or washability of the product will be greatly enhanced. Where such a hygiene product was treated with antimicrobial agents, the inventor found out that a surface can be sanitized by wiping the textile material over the surface.

Sanitizing a surface by wiping a textile material preferably implies a reduction of at least certain types of living bacteria such as *Escherichia coli* ATCC 25922 and/or *Staphylococcus aureus* ATCC 6538 and/or *Pseudomonas aeroginosa* ATCC 15442 and/or *Salmonella enterica* ATCC 10708, preferably most types of living bacteria, more preferably all types of living bacteria, and/or a reduction of *Candida albicans* ATCC 10231 and/or *Aspergillus niger* 16404 on the surface by at least 99% (2 log), preferably at least 99.9% (3 log), more preferably at least 99.99% (4 log), even more preferably at least 99.999% (5 log), particularly at least 99.9999% (6 log), preferably evaluated after 0, 15, and/or 60 minutes post wiping.

Sanitizing a surface may result in at least 50%, preferably at least 90% (1 log), more preferably at least 99% (2 log), even more preferably at least 99.9% (3 log), particularly at least 99.99% (4 log) or 99.999% (5 log), and most preferably at least 99.9999% (6 log) of one or more or preferably all types of living bacteria and/or other microbes picked up by the textile material being killed. The killing occurs preferably on the textile material, and more preferably by the antimicrobial agents adhered to the textile material, preferably within 60 minutes, more preferably within 30 minutes, even more preferably within 15 minutes post wiping. In this case, cross-contamination of other surfaces or humans by touching the textile material after use can be avoided. Furthermore, fouling of the textile material and the development of odors is prevented even if the textile material is not washed and/or dried after use.

The surfaces can even be sanitized as described above if no liquid antimicrobial agent is applied to the surface and/or wherein at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material is released from the textile material onto the surface.

With some textile materials finished according to the invention, where liquid antimicrobial agent is applied to the surface and/or wherein more than 1% of an antimicrobial agent, in particular of a liquid antimicrobial agent, adhered to, contained in or held by the textile material is released from the textile material onto the surface, the sanitizing would be achieved even if no liquid antimicrobial agent was used and/or if at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material was released from the textile material onto the surface.

In some embodiments, the textile material and/or the surface is wetted prior to wiping the textile material over the surface to be sanitized. However, the inventor found that surprisingly, surfaces can even be sanitized with textile materials manufactured according to the invention if the textile material is dry during wiping it over the surface.

Wiping the textile material over the surface to sanitize the surface is performed at least once, for at least 5 seconds, for at least 10 seconds, for at least 30 seconds, for at least 60 seconds, or for at least 90 seconds. In some uses, wiping the textile material over the surface is performed for at most 90 seconds, for at most 60 seconds, for at most 30 seconds, for at most 10 seconds, or only once.

During wiping the textile material over the surface, an average pressure of at least 5 g/cm², preferably at least 10 g/cm², more preferably at least 25 g/cm², even more preferably at least 50 g/cm², particularly at least about 120 g/cm² may be exercised on at least parts of the textile by a hand of the user or other wiping tool, and/or a maximum pressure of at most 500 g/cm², preferably at most 400 g/cm², more preferably at most 300 g/cm², most preferably at most about 200 g/cm². Furthermore, an average pressure of at most 600, preferably at most 500 g/cm², more preferably at most 400 g/cm², particularly at most 300 g/cm², and most preferably at most 200 g/cm² may be exercised on at least parts of the textile by a hand of the user or other wiping tool.

## Claims

1. Textile material to which one or more antimicrobial agents and one or more stain release agents are adhered,
the textile material exhibiting a water absorbency time of at most 5 seconds, preferably at most 3 seconds, more preferably at most 2 seconds, and most preferably at most 1 second, preferably when measured in accordance with AATCC test method 79-2014 (Option A).

2. The textile material of claim 1, wherein the one or more antimicrobial agents adhered to the textile material comprise
- at least one, preferably at least two, more preferably at least three, even more preferably at least four, and most preferably all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably at least three, and most preferably all four selected from the group consisting of metal, an azole-based compound, a quaternary ammonium organosilane compound, and polyhexamethylene biguanide; or
- at least one, preferably at least two, more preferably all three selected from the group consisting of metal, an azole-based compound, and polyhexamethylene biguanide ; or
- at least one, preferably at least two, more preferably all three selected from the group consisting of a quaternary ammonium organosilane compound, metal, and an azole-based compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

3. The textile material of any one of the preceding claims, wherein the one or more antimicrobial agents adhered to the textile material increase the reduction value of the textile material and/or the textile material exhibits a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 measured in accordance with AATCC test method 100-2012 and/or ASTM E2149-10 by/of at least 90% (1 log), preferably at least 99% (2 log), more preferably at least 99.9% (3 log) within 10 minutes of contact time and/or of at least 99% (2 log), preferably at least 99.9% (3 log), more preferably at least 99-99% (4 log) within 1 hour of contact time and/or of at least 99.% (2 log), preferably at least 99.9% (3 log), more preferably at least 99.99% (4 log), most preferably at least 99.999% (5 log) within 24 hours of contact time.

4. The textile material of any one of the preceding claims, wherein when sanitizing a ceramic surface by wiping a textile material over the surface, wherein no liquid antimicrobial agent is applied to the surface and/or wherein at most 1%, preferably at most 0.1%, more preferably at most 0.01%, particularly at most 0.001%, and most preferably at most 0.0001% of any antimicrobial agent, in particular of any liquid antimicrobial agent, adhered to, contained in or held by the textile material is released from the textile material onto the surface, a reduction of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 on the surface by at least 99% (2 log), preferably at least 99.9% (3 log), more preferably at least 99.99% (4 log), even more preferably at least 99.999% (5 log), particularly at least 99.9999% (6 log), preferably evaluated after 0, 15, and/or 60 minutes post wiping can be achieved, preferably using ISO test method 18539:2004.

5. The textile material of claim 4, wherein the reduction can be achieved when the textile material is dry during wiping it over the surface.

6. The textile material of any one of the preceding claims, wherein the one or more stain release agents adhered to the textile material comprise at least one selected from the group consisting of fatty alcohol ethoxylates and organosilane terpolymers, preferably organosilane terpolymers.

7. The textile material of any one of the preceding claims, wherein the one or more stain release agents adhered to the textile material increase the stain release rating of the textile material by at least one grade, more preferably by at least two grades, particularly at least three grades, and most preferably four grades, when tested according to AATCC test method 130-2010.

8. The textile material of any one of the preceding claims, having a stain release rating, measured in accordance with AATCC test method 130-2010, of at least grade 3, preferably at least grade 4, and most preferably grade 5.

9. The textile material of any one of the preceding claims, wherein the claimed properties of the textile material, such as stain release capability, antimicrobial efficiency, and/or properties related to hydrophilicity are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

10. A process for finishing a textile material, comprising the steps of:
- treating the textile material using an antimicrobial liquor application process like an exhaustion or padding process, wherein the liquor comprises one or more antimicrobial agents;
- drying the textile material and treating the textile material using a stain release liquor application process like an exhaustion or preferably a padding process, wherein the liquor comprises one or more stain release agents; and
- subjecting the treated textile material to a heat treatment.

11. The process for finishing a textile material of claim 10, wherein the antimicrobial liquor application process is preferably an exhaust process, and between the step of treating the textile material using an antimicrobial liquor application process and the steps of drying the textile material and treating the textile material using a stain release liquor application process, one or more process cycles are performed, comprising the steps of drying the textile material and treating the textile material using a further antimicrobial liquor application process, preferably a padding process, wherein the liquor comprises one or more antimicrobial agents.

12. The process of any one of claims 10 to 11, wherein the heat treatment comprises curing of the textile material, wherein the curing is conducted at least partially at a curing ambient temperature of at least 140 °C, preferably at least 160 °C, more preferably at least 170 °C, particularly at least 175 °C, and most preferably at least about 180 °C.

13. The process of any one of claims 10 to 12, wherein, where an antimicrobial and/or hydrophilic/stain release liquor application process is an exhaustion process, the liquor has a temperature of at least 45 °C, preferably of at least 50 °C, more preferably of at least 60 °C, particularly of at least 70 °C, and most preferably of at least about 80 °C.

14. The process of any one of claims 10 to 13, wherein the one or more antimicrobial agents are selected as defined in claim 2 and/or the one or more stain release agents are selected as defined in claim 6.

15. A method of sanitizing a surface by wiping a textile material according to any of claims 1 to 14 over the surface, wherein sanitizing means a reduction of at least certain types of living bacteria such as *Escherichia coli* ATCC 25922 and/or *Staphylococcus aureus* ATCC 6538 and/or *Pseudomonas aeroginosa* ATCC 15442 and/or *Salmonella enterica* ATCC 10708, preferably most types of living bacteria, more preferably all types of living bacteria, and/or a reduction of *Candida albicans* ATCC 10231 and/or *Aspergillus niger* 16404 on the surface by at least 99% (2 log), preferably at least 99.9% (3 log), more preferably at least 99-99% (4 log), even more preferably at least 99.999% (5 log), particularly at least 99.9999% (6 log), preferably evaluated after 0, 15, and/or 60 minutes post wiping.
